# EUROPEAN PATENT APPLICATION

(11) **EP 4 364 646 A1**
(43) Date of publication of application: **08.05.2024**
(21) Application number: 22205519.6
(22) Date of filing: 04.11.2022
(51) Int. Cl.: A61B 5/00, A61N 1/36, A61B 5/055

(54) **MIGRAINE MANAGEMENT USING FUNCTIONAL BRAIN IMAGING**

(71) Applicant: Koninklijke Philips N.V., 5656 AG Eindhoven (NL)
(72) Inventor: MENA BENITO, Maria Estrella, Eindhoven (NL); VAN EE, Raymond, Eindhoven (NL); LEUFKENS, Timmy Robertus Maria, 5656AG Eindhoven (NL)
(74) Representative: Philips Intellectual Property & Standards

(57) **Abstract**

The present invention relates to a device (20) for analyzing neural activity in the brain of a patient by functional brain imaging to diagnose, assess, follow-up and/or classify a migraine condition of the patient. The device comprises an input (21) for receiving functional brain imaging data of the brain of the patient and an an output (22) for outputting at least one value. Furthermore, the device comprises a processor (23) for determining at least one activity measure indicative of brain activity in at least one predetermined region of interest, and/or between such regions of interest, based on the functional brain imaging data. The processor is adapted to determine and output the at least one value based on (at least) said activity measure(s), The value(s) represent(s) a patient-specific migraine score, a migraine type classification, a prognostic/predictive value, an assessment of influence of different types of migraine triggering events and/or of efficacy and/or suitability of different treatments.

## Description

### FIELD OF THE INVENTION

The present invention relates to the field of medical imaging, and, more specifically, the invention relates to methods, devices, systems and computer-program products for migraine assessment, diagnosis and/or treatment support by functional brain imaging.

### BACKGROUND OF THE INVENTION

Migraine is considered to be, generally, quite a costly neurological disease in view of its impact, and is even the third most prevalent disorder in well-developed countries such as in the European Union and the United States. Furthermore, it is the sixth most common specific cause of disability worldwide. Migraine is a medical disorder that is typically associated with a pounding, throbbing pain on one side of the head. While most migraines last about four hours (or less), some can last much longer, e.g. even up to 72 hours.

Migraine headache attacks are often accompanied by functionally and structurally maladaptive changes in the brain. Several studies have reported that migraine, as a dysfunctional brain state, probably relates to long-lasting anatomical and functional effects.

The pain due to migraine attacks can be so debilitating that the inflicted patient is often not able to perform any meaningful activities at all, and can thus have serious consequences for quality of life of the individual and society at large in view of the prevalence of this condition. Besides headaches, migraines can also be associated with nausea and/or dizziness. Furthermore, patients are often very sensitive to bright lights and/or loud noises. Severe sensory hypersensitivity may particularly occur during migraine attacks, and/or may indicate the onset of an attack, possibly even acting as a cause or facilitating factor of the attack.

Migraine episodes are known to develop over different, distinct phases. However, not all phases are necessarily evident in all patients or in every migraine attack, adding to the complexity of diagnosis and management of the condition. For example, migraines may be preceded or accompanied by sensory warning symptoms, referred to as aura, such as flashes of light, blind spots, or tingling sensations in the extremities, e.g. in an arm or leg. The perception of other senses can be affected as well, before or during the migraine attack. For example, the patient, also referred to as the migraineur, may sense strange smells or tastes. It is also known that the individual attack frequency and severity can be strongly influenced by individual health and life-style factors. Nonetheless, migraine currently remains a relatively poorly understood condition.

Migraines can be classified based on the frequency of attacks, e.g. based on how often attacks happen in a month. If a person suffers from migraine symptoms for less than fifteen days, the migraine is generally considered to be episodic in nature, whereas symptoms that persist after fifteen days are indicative of a chronic migraine condition. For the quality of life of migraineurs, an accurate diagnosis of the migraine, and particularly the prediction and/or detection of migraine attacks, forms an important and crucial challenge.

The success of migraine treatments varies from person to person due to difference in specific individual factors and also the patient's experiences of migraine pain. Adding to the complexity of an accurate diagnosis, some people suffering from migraine may show less common symptoms, e.g. other than the typically to-be-expected headache.

Distinguishing between the various causes and types of migraine seems to be crucial for an accurate diagnosis. Currently, diagnosis of migraine relies heavily on medical history and self-reporting diaries. The current reliance on such migraine diaries, in which the migraineur reports the course of migraine attacks and non-migraine periods, may not offer the most appropriate tool to achieve the required diagnostic accuracy. For example, when such information is gathered over a sufficiently long time, conclusions as to the nature and possible patterns of the recurrent migraine attacks may be drawn therefrom. However, self-reporting can be impractical, is often not very accurate and is, obviously, rather subjective. Therefore, many migraine patients may not receive the correct diagnosis and/or may not receive the most suitable treatment, e.g. appropriate medication, due to the difficulties in establishing an accurate and objective diagnosis.

A need exists in the art for accurate means and methods for objective measuring relevant parameters to assess and diagnose migraine. Functional imaging methods may have a potential for use in defining biomarkers for diagnostic and treatment purposes, e.g. a need exists for functional imaging measures that are selectively sensitive to migraine as opposed to other headaches.

Furthermore, the medication known in the art is often only moderately effective and may be poorly tolerated. A poor understanding of the migraine attacks, the type of migraine, its severity and its frequency may form important limiting factors to the efficacy of a treatment plan. Overuse of painkillers may also cause serious side effects, such as comorbid depression, stress, and obesity, and forms a recognized risk factor for chronification.

Various functional brain imaging studies have attempted to improve our understanding of migraine and its possible mechanisms. For example, positron emission tomography (PET) and magnetic resonance imaging (MRI) studies have provided insights into migraine mechanisms and have identified brain regions that might be responsible for mediating the onset of migraine attacks. For example, while migraine is commonly associated with cognitive impairment, other types of headaches, such as tension type headache and cluster headaches, are not associated with cognitive impairment as such, i.e. at least during the headache-free periods.

### SUMMARY OF THE INVENTION

It is an object of embodiments of the present invention to provide in good, efficient and/or accurate means and methods for migraine assessment, classification and/or diagnosis, e.g. for use in a personalized management approach for migraine.

It is an advantage of embodiments of the present invention that the clinical management of migraine can be personalized, e.g. optimized, for each specific patient.

It is an advantage of embodiments of the present invention that objective measures can be used to assess and/or diagnose migraine, e.g. to reduce or overcome the limitations of diagnosis by means of medical history and/or subjective self-reporting.

It is an advantage of embodiments of the present invention that an accurate and/or robust diagnosis of migraine can be achieved. For example, by providing means and/or methods that can distinguish different sources and/or types of migraine, a more accurately finetuned diagnosis and/or therapy may be defined.

It is an advantage of embodiments of the present invention that migraine can be accurately and objectively distinguished from other conditions associated with headaches, and/or that different types of migraine can be objectively distinguished, e.g. that a migraine condition can be correctly classified.

It is an advantage of embodiments of the present invention that individual differences in migraine symptoms, episode frequency and/or severity can be taken into account and/or confounding effects, such as the influence of individual health and/or lifestyle factors, can be reduced and/or accounted for.

It is an advantage of embodiments of the present invention that the influence of individual differences in the perception of pain between patients on the diagnosis and treatment of migraine can be reduced, e.g. that the reliance on self-reporting in diagnosis can be reduced, replaced and/or augmented by quantitative data.

It is an advantage of embodiments of the present invention that migraine management can be personalized and customized at the level of the individual patient in a robust and quantifiable manner.

It is an advantage of embodiments of the present invention that imaging data of a patient can be combined with longitudinal patient data in a simple measure indicative of migraine, e.g. an individual migraine index. Thus, a quantifiable metric is provided that allows the effect of migraine on the specific patient to be followed up and assessed accurately in a simple manner.

It is an advantage of embodiments of the present invention that the measure thus provided can be used for clinical support, e.g. to classify the type and nature of a migraine condition at the level of the individual patient, to assess its severity and/or to improve migraine management by tailoring a therapy to the patient's needs and response.

In a first aspect, the present invention relates to a method, e.g. a computer-implemented, method for analyzing neural activity in the brain of a patient by functional brain imaging to diagnose, assess, follow-up and/or classify a migraine condition of the patient. The method comprises acquiring functional brain imaging data (e.g. one or more images of the patient's brain, e.g. tomographic images, e.g. volumetric 3D images). The imaging data may for example be acquired in the interictal phase of the migraine (e.g. in the absence of present migraine symptoms) and/or in the ictal phase (e.g. while migraine symptoms are presenting, e.g. during a migraine attack, and/or shortly before/after the attack).

The method comprises determining at least one activity measure indicative of brain activity in at least one predetermined region of interest in the patient's brain, and/or between predetermined regions of interest (e.g. correlated activity) in the patient's brain, based on said functional brain imaging data.

The method comprises determining and outputting at least one value, based on said at least one activity measure, that is representative of a patient-specific migraine score indicating a probability and/or severity of the migraine, a migraine type classification, a prognostic value to predict frequency of migraine attacks and/or an estimated time until a next attack and/or a future evolution of the migraine condition, and/or an assessment of the influence of different types of migraine triggering events and/or of the predicted efficacy and/or suitability of different treatments.

In a method in accordance with embodiments, the determined at least one activity measure may be representative of brain activity in, and/or expresses a relationship of local activity within and/or between one or more of, said at least one predetermined region of interest. The at least one predetermined region of interest may comprise a brain region or brain regions involved in pain signal processing and/or pain sensation, attentional processing, executive function, sensory processing and/or sensorimotoric function, resting wakeful state maintenance, memory function and/or emotion.

A method in accordance with embodiments may comprise receiving further imaging, physiological, medical, psychometric, behavioral, lifestyle, self-reported pain experience and/or diagnostic data pertaining to said patient and taking said further data into account in determining the activity measure and/or in determining the at least one value for output.

In a method in accordance with embodiments, acquiring the functional brain imaging data may be repeated at different times, e.g. separated by at least a day or at least a month or at least 3 months, so as to determine an evolution over time (e.g. to assess the condition substantially over the long term) by the correspondingly determined activity measures (and/or output values determined for each individual session) and to determine and output at least one (e.g. further) value representative of changes in the migraine condition over time and/or representative of a future prediction of changes in the migraine condition.

In a method in accordance with embodiments, acquiring the functional brain imaging may comprise providing a plurality of stimuli at different points in time via at least one stimulus source to the patient during the acquisition of said functional brain imaging data to induce (and/or affect) said brain activity (e.g. directly or indirectly).

In a method in accordance with embodiments, said plurality of stimuli may be provided in accordance with a blocked-trial functional imaging paradigm and/or an event-related functional imaging paradigm. Determining the at least one activity measure may comprise processing the functional brain imaging data in accordance with said paradigm (e.g. using information regarding the timing of the stimuli in processing the imaging data). In a method in accordance with embodiments, determining the at least one activity measure may comprise using a model to analyze the functional imaging data so as to determine the at least one activity measure (e.g. a strength of activation and/or a statistical significance of activation) as explained by the plurality of stimuli. The model may for example comprise a general (or generalized) linear model (GLM), e.g. using a statistical parametric mapping (SPM) approach, and/or may comprise evaluating a trained machine learning model.

In a method in accordance with embodiments, said plurality of stimuli may comprise nociceptive stimuli, and the at least one predetermined region of interest may comprise one or more regions of the pericentral sensorimotor network and/or one or more brain regions affected by, controlling, contributing to and/or modulating physiological and/or behavioral responses to pain, so as to detect an imbalance in brain activation between pain-facilitation and pain-inhibition in the brain.

In a method in accordance with embodiments, the at least one predetermined region of interest may comprise any one, or combination of, the amygdala, the insula, the temporal pole, the brainstem, the medulla oblongata, the spinal trigeminal nucleus, the subnucleus caudalis, the midbrain tegmentum, the periaqueductal gray, the dorsal pons, the lentiform nuclei, the thalamus, the posterior thalamus, the ventral posteriomedial nucleus, the fusiform gyrus, the cerebellum, the subthalamic nucleus, the hippocampus, the parahippocampus, the hypothalamus, the cingulate cortex, the prefrontal cortex, the somatosensory cortex, the pre- and/or post-central gyrus, the dorsolateral prefrontal cortex, the superior temporal gyrus and/or a subregion or subregions of any of the aforementioned regions.

In a method in accordance with embodiments, the plurality of stimuli may comprise stimuli in accordance with a Posner cueing task and/or (e.g. another) attentional processing task, wherein the at least one predetermined region of interest may comprise one or more regions of the midcingulo-insular salience network to detect facilitated hypersensitivity by dysfunctional suppression of non-attended events and/or other dysfunctional attentional processing in the selection of, mental shifting between and/or continued focus maintenance on attended versus unattended events provided in said task.

In a method in accordance with embodiments, the plurality of stimuli may comprise stimuli provided in accordance with a cognitive processing task, in which the at least one predetermined region of interest may comprise one or more regions of the frontoparietal and/or lateral fronto-parietal central executive network.

In a method in accordance with embodiments, the plurality of stimuli may comprise visual stimuli, auditory stimuli, tactile stimuli, olfactory stimuli, thermal stimuli and/or other sensory stimuli.

In a method in accordance with embodiments, providing the plurality of stimuli may comprise providing different sets of sensory stimuli, corresponding to different sensory stimulation conditions (e.g. different sensory modalities, content types, ...) and/or different stimulation characteristics (e.g. parameters, such as a stimulation strength, frequency, ...). The at least one activity measure and/or the at least one output value may be determined so as to score and/or rank the different sets of sensory stimuli and/or related stressors for their estimated potential to evoke, facilitate and/or trigger a migraine attack.

In a method in accordance with embodiments, the plurality of stimuli may comprise visual stimuli, and the at least one predetermined region of interest may comprise one or more regions of the brain involved in vision so as to detect abnormal activity in, and/or interaction within and/or between, the visual cortex, the thalamus and/or other regions involved in the processing of visual information.

In a method in accordance with embodiments, the at least one activity measure, or component thereof, in response to visual stimuli may be used to determine the at least one output value, or component thereof, for classifying the patient as a migraine-with-aura or migraine-without-aura patient and/or to quantify a hypersensitivity to visual stimuli.

In a method in accordance with embodiments, acquiring the functional brain imaging data may comprise acquiring functional imaging data in a resting state of the patient, without concomitant external stimulation, and determining the at least one activity measure may comprise performing a resting-state functional connectivity imaging analysis to determine functional connectivity within and/or between said at least one predetermined region of interest, in which the at least one predetermined region of interest may comprise one or more regions of the default mode network, the midcingulo-insular salience network and/or the fronto-parietal central executive network.

A method in accordance with embodiments may comprise determining at least one morphometric value of one or more of said at least one predetermined region of interest based on said functional brain imaging data and/or further imaging data of the patient's brain, and determining (14) the at least one activity measure and/or the at least one output (15) value may take said at least one morphometric value into account.

In a method in accordance with embodiments, acquiring the functional brain imaging data may comprise acquiring single photon emission, positron emission tomography images, magnetic electroencephalography images, electro-encephalography images, and/or any other suitable functional brain imaging modality, and/or any combination thereof.

In a method in accordance with embodiments, acquiring the functional brain imaging data may comprise acquiring a timeseries of functional magnetic resonance images of the brain of the patient using a magnetic resonance imaging system.

In a second aspect, the present invention relates to a device, e.g. a computer system and/or specifically adapted and/or programmed processing hardware, e.g. a device for executing a method in accordance with embodiments of the first aspect of the present invention. The device is adapted to analyze neural activity in the brain of a patient by functional brain imaging to diagnose, assess, follow-up and/or classify a migraine condition of the patient. The device comprises an input for receiving functional brain imaging data, e.g. one or more images of the patient's brain, e.g. tomographic images, e.g. volumetric 3D images. The device comprises an output for outputting at least one value, and a processor for determining at least one activity measure indicative of brain activity in and/or between at least one predetermined region of interest in the patient's brain based on said functional brain imaging data. The processor is furthermore adapted to determine and output said at least one value, via said output, based on the at least one activity measure, This at least value is representative of a patient-specific migraine score indicating a probability and/or severity of the migraine, a migraine type classification, a prognostic value to predict frequency of migraine attacks and/or an estimated time until a next attack and/or a future evolution of the migraine condition, and/or an assessment of the influence of different types of migraine triggering events and/or of the predicted efficacy and/or suitability of different treatments.

In a device in accordance with embodiments, the processor may be adapted to determine said at least one activity measure representative of brain activity in, and/or expresses a relationship of local activity within and/or between one or more of, said at least one predetermined region of interest, wherein the at least one predetermined region of interest comprises a brain region or brain regions involved in pain signal processing and/or pain sensation, attentional processing, executive function, sensory processing and/or sensorimotoric function, resting wakeful state maintenance, memory function and/or emotion.

A device in accordance with embodiments may comprise a further input for receiving further imaging, physiological, medical, psychometric, behavioral, lifestyle, self-reported pain experience and/or diagnostic data pertaining to said patient, in which the processor is adapted to take the further data into account in determining the activity measure and/or in determining the at least one value.

A device in accordance with embodiments may comprise a further output for controlling at least one stimulus source (which may be external and/or included in the device). The processor may be adapted for providing a plurality of stimuli to the patient at different points in time via the further output and the at least one stimulus source during acquisition of said functional brain imaging data so as to induce said brain activity.

In a device in accordance with embodiments, the processor may be adapted to provide said plurality of stimuli in accordance with a blocked-trial functional imaging paradigm and/or an event-related functional imaging paradigm, and for determining the at least one activity measure by processing the functional brain imaging data in accordance with said paradigm using a model for taking the timing of said plurality of stimuli and of the acquisition of said functional brain imaging data into account.
In a device in accordance with embodiments, the processor may be adapted to determine said at least one activity measure using said model, wherein the model comprises a general or generalized linear model, a statistical parametric mapping model and/or a trained machine learning model. Additionally or alternatively, the at least one value may be determined by a trained machine learning model, e.g. based on the at least one activity measure, or directly from the functional brain imaging data (or intermediate features derived therefrom). In other words, the activity measure may also be implicitly encoded in an intermediate stage of the machine learning model.

A device in accordance with embodiments may comprise the at least one stimulus source, in which the at least one stimulus source may be adapted for providing the plurality of stimuli to the patient and my comprise a nociceptive stimulator for generating nociceptive stimuli, a sound source for generating auditory stimuli, a light source and/or display for generating visual stimuli, a haptic and/or tactile stimulator for generating haptic stimuli, an olfactory stimulator for generating olfactory stimuli, a heater and/or cooler for generating thermal stimuli and/or a sensory stimulator to generate other sensory stimuli.

In a device in accordance with embodiments, the processor may be adapted to provide the plurality of stimuli, comprising nociceptive stimuli, and to determine the at least one activity measure indicative of the brain activity in and/or between the at least one predetermined region of interest comprising one or more regions of the pericentral sensorimotor network and/or one or more brain regions affected by, controlling, contributing to and/or modulating physiological and/or behavioral responses to pain, so as to detect an imbalance in brain activation between pain-facilitation and pain-inhibition.

In a device in accordance with embodiments, the processor may be adapted to provide the plurality of stimuli in accordance with a Posner cueing task and/or another attentional processing task, and to determine the at least one activity measure indicative of the brain activity in and/or between the at least one predetermined region of interest comprising one or more regions of the midcingulo-insular salience network to detect facilitated hypersensitivity by dysfunctional suppression of non-attended events and/or other dysfunctional attentional processing in the selection of, mental shifting between and/or continued focus maintenance on attended versus unattended events provided in said task.

In a device in accordance with embodiments, the processor may be adapted to provide the plurality of stimuli in accordance with a cognitive processing task, and to determine the at least one activity measure indicative of the brain activity in and/or between the at least one predetermined region of interest comprising one or more regions of the frontoparietal and/or lateral fronto-parietal central executive network.
In a device in accordance with embodiments, the processor may be adapted to provide different sets of sensory stimuli at different times and/or in different combinations, wherein said different sets correspond to different sensory stimulation conditions and/or stimulation characteristics, and wherein said processor is adapted to determine the at least one activity measure and/or the output value so as to score and/or rank the different types of sensory stimuli and/or thereto related stressors for their estimated potential to evoke, facilitate and/or trigger a migraine attack.

In a device in accordance with embodiments, the processor may be adapted to provide the plurality of stimuli, comprising visual stimuli, and to determine the at least one activity measure indicative of the brain activity in and/or between the at least one predetermined region of interest comprising one or more regions of the visual cortex, the thalamus and/or other brain areas involved in the processing of visual information and/or vision, so as to detect abnormal activity in, and/or interaction within and/or between said one or more regions.

In a device in accordance with embodiments, the at least one activity measure, or component thereof, in response to visual stimuli may be used (by the processor) to determine the at least one output value, or component thereof, for classifying the patient as a migraine-with-aura or migraine-without-aura patient.

In a device in accordance with embodiments, the processor may be adapted to receive, via said input, said functional brain imaging data comprising functional imaging data acquired in a resting state of the patient, without concomitant external stimulation, and may be adapted to determine the at least one activity measure, or component thereof, by performing a resting-state functional connectivity imaging analysis so as to determine functional connectivity within and/or between said at least one predetermined region of interest, wherein the predetermined region of interest comprises one or more regions of the default mode network, the midcingulo-insular salience network and/or the fronto-parietal central executive network.

In a device in accordance with embodiments of the present invention, the processor may furthermore be adapted to determine at least one morphometric value of one or more of said at least one predetermined region of interest based on said functional brain imaging data and/or on further imaging data of the patient's brain, and to take said at least one morphometric value into account in determining the at least one activity measure and/or the at least one output value.

In a third aspect, the present invention relates to a magnetic resonance imaging system adapted to perform a method in accordance with embodiments of the first aspect of the present invention, and/or comprising a device in accordance with embodiments of the second aspect of the present invention.

In a fourth aspect, the present invention relates to a computer-program product adapted to perform a method in accordance with embodiments of the first aspect of the present invention when executed by a computer, e.g. for implementing, by execution thereof, a device in accordance with embodiments of the second aspect of the present invention.

The independent and dependent claims describe specific and preferred features of the invention. Features of the dependent claims can be combined with features of the independent claims and with features of other dependent claims as deemed appropriate, and not necessarily only as explicitly stated in the claims.

### BRIEF DESCRIPTION OF THE DRAWINGS

Fig. 1 shows a magnetic resonance imaging system in accordance with embodiments of the present invention.
Fig. 2 illustrates a method in accordance with embodiments of the present invention.
Fig. 3 schematically shows a device in accordance with embodiments of the present invention.

The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a method for analyzing neural activity in the brain of a patient by functional brain imaging to diagnose, assess, follow-up and/or classify a migraine condition, e.g. to assist a clinician in diagnosing the migraine condition, correctly classify a type of migraine, quantify the severity of a migraine case, assess the effectiveness of a therapy and/or define (or finetune) a suitable therapy plan. Thus, embodiments of the present invention may contribute to, e.g. may enable, a personalized management approach for migraine. Given the state of the present knowledge regarding this condition and current clinical practice methodology, it will be understood that a method to improve the differential diagnosis, classification and treatment of migraine based on objective measures may provide important advantages and, ideally, could lead to a more effective management of the condition and/or to improved clinical outcomes for migraine patients.

The functional brain imaging may comprise functional magnetic resonance imaging (fMRI), positron emission tomography (PET), magnetic electroencephalography (MEG), electro-encephalography (EEG), any other suitable functional brain imaging modality, and/or any combination of such functional brain imaging modalities.

The method may particularly be a computer-implemented method, e.g. performed by a computer program product when executed on a suitable computing platform. For example, the method may be implemented by software for a suitable computing platform, e.g. for a programmable microprocessor and/or general-purpose computer, and/or by hardware, e.g. digital signal processing hardware.

The method may be adapted to monitor and/or determine the effect of stimuli on a patient, to evaluate the evoked physiological effect of such stimuli by functional imaging, to quantify these effects, and/or to determine one or more measures, e.g. a score or rating, expressing (a) characteristic(s) of the migraine condition, such as a probability (or related measure, e.g. likelihood) that the patient suffers from migraine, a classification into different types of migraine condition (e.g. a score for each possible class), a measure indicative of the severity of the migraine, a predictive measure of frequency of migraine attacks and/or of time until a next attack. This may also include a score or rating (or similar indicative measure) for different treatment modalities to predict their likely efficacy and/or suitability, e.g. of different drugs, lifestyle interventions, therapeutical approaches, and/or combination treatments. Thus, a personalized therapy plan may also be generated based on such outputs, e.g. as a computer-aided suggestion to the attending physician.

The method may also rely on inherent functional relationships detected in the imaging data, e.g. without necessarily requiring stimuli. For example, resting state functional (connectivity) magnetic resonance imaging (rs-fcMRI) may be used, as known in the art, to detect regional correlations between (and/or within) regions of the brain, e.g. in a functional network analysis. Other measures for expressing functional features of brain regions and/or their intra- and/or interregional connectivity, e.g. based on resting state fMRI, may be used as well (additionally or alternatively), such as, for example, regional homogeneity (ReHo) of the fMRI signal in resting state and/or discriminative analyses applied to rs-fcMRI data, e.g. for differentiating migraineurs from healthy subjects (and/or different classes of migraine patients) based on (e.g.) classification features of the fMRI data.

Embodiments of the present invention may also combine a stimulation-based protocol (e.g. block-design and/or event-related fMRI) with non-stimulation-based functional imaging (e.g. rs-fcMRI). It will be understood that such 'stimulated' and 'non-stimulated' image acquisition can be easily combined in an integrated (i.e. joint) imaging protocol, e.g. can be performed within a single imaging session (without limitation thereto).

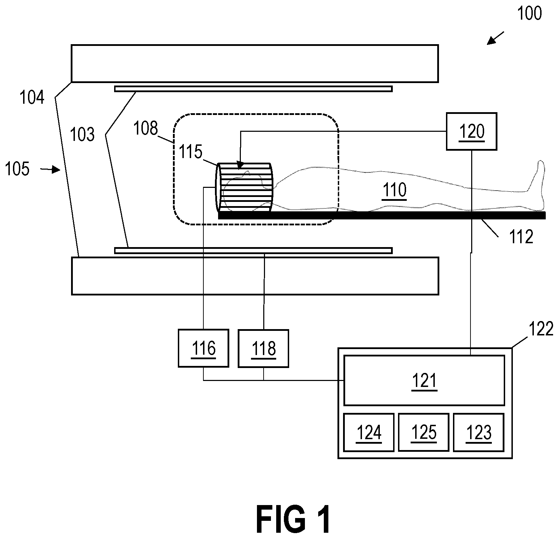

The method may be performed by interacting with a magnetic resonance imaging system. Fig. 1 shows an example of such magnetic resonance imaging system 100, e.g. an MRI system that is generally suited to implement steps of a method in accordance with embodiments of the present invention. In a further aspect, the present invention relates to such MRI system 100 adapted to perform a method in accordance with embodiments of the first aspect of the present invention.

The MRI system 100 comprises a magnet 104, e.g. a superconducting cylindrical magnet through which a bore 105 is defined. Variations of and/or alternatives for this type of magnet are also possible, e.g. the magnet may be a split cylindrical magnet or an open magnet (e.g. in which magnet sections are arranged around, e.g. above and below, an open space for receiving the subject to be imaged). The magnet 104 may comprise a plurality of superconducting coils, e.g. in a cryostat. Inside the bore 105, an imaging region 108 is defined in which a subject can be imaged. In this imaging region 108, the magnetic field of the magnet 104 is typically strong and uniform, i.e. sufficiently strong and uniform to perform MR imaging. For an open magnet or other alternative magnet assembly, there might not be a bore cavity defined, but the imaging region 108 will still be clearly identifiable, e.g. as characterized by a suitable magnetic field strength and homogeneity. The magnetic resonance data acquired by the system, in use thereof, is typically representative of the content of the space defined by the imaging region 108, or a part thereof, e.g. a specific region of interest defined inside this imaging region.

A patient 110 can be, in use, placed inside the imaging region 108. For this, the system may comprise a subject support 112, e.g. an actuated patient couch. By controlling the subject support, a body part or region of interest in the subject on the support can be moved to a suitable position inside the imaging region 108.

The system may further comprise a plurality of magnetic field gradient coils 103, adapted to spatially encode magnetic spins within the imaging region 108 (e.g. by superimposing local magnetic field variations onto the strong magnetic field of the magnet). For example, the magnetic field gradient coils may comprise at least three coils (or sets of coils) to allow spatial encoding in three orthogonal spatial directions, i.e. in any direction in space. The magnetic field gradient coils 103 may be controlled as a function of time, in addition to control such as to select the spatial encoding direction, e.g. to create gradients that are pulsed, ramped or otherwise varying as function of time. For example, a power supply of the magnetic field gradient coils may be controlled by a controller, processor or computer to achieve a magnetic field (gradient) in accordance with a desired spatial and/or temporal function. The system may typically comprise a controller 118 to control the magnetic field gradient coils 103.

The system may typically comprise a radiofrequency (RF) coil 115 (an RF antenna device) which may act as a radiofrequency receiver and transmitter. For example, the RF coil may transmit a signal that disrupts the alignment of (nuclear) spins with the magnetic field (of atoms and molecules in the imaging region 108), and the RF coil may receive a return signal that is generated by a precession and relaxation of the disrupted nuclear spins back to the magnetic field alignment. The RF coil may be a construct comprising a plurality of separate coil elements, and/or support equipment. The RF coil may be integrated in the bore system, and/or may be a separate component to be placed close to or in contact with the imaged patient. For example, the RF coil(s) may be integrated in a head coil assembly for imaging the brain of the subject 110.

The RF coil 115 comprises, or is connected to, an RF transceiver 116. Obviously, the system may also comprise separate RF transmission and receival antennas and corresponding (separate) receiver and transmitter systems, or any combination of a plurality of antenna coils and transceiver and/or receiver and/or transmitter systems. For example, a plurality of antenna coils may be connected to separate receive and/or transmit channels, which may be controlled separately, in unison or in any combination that is considered suitable for the intended purpose. A number of parallel channels may, for example, be useful for parallel imaging techniques, such as SENSE or ASSET (sensitivity encoding; array coil spatial sensitivity encoding).

The MRI system 100 may also comprise a stimulus source 120 to provide a stimulus to the patient during an MRI imaging operation. The stimulus source(s) 120 specifically comprises one or more sensory stimulus sources, to provide a signal (stimulus/stimuli) to the patient via any of (or combination of) the physical senses of the body, such as nociception, touch, smell, taste, hearing and sight. The sensory stimulus source may include a light(s) and/or display to present a visual stimulus to the patient while positioned in the imaging region (e.g. including possibly a projection screen and/or optical system to relay displayed information to within the bore volume), and/or a speaker or other sound source to present an auditory stimulus to the subject (including possibly a relay system, e.g. using guided air pressure transmission or contact conduction of sound into the bore volume, e.g. to a headphone). Other examples include means to provide a somatosensory stimulus to the patient, such as a haptic/tactile signal, a system to control a (perceivable) directed airflow onto the body of the patient, a device to deliver a pain signal to the body (e.g. via release of a noxious chemical, e.g. ammonia, by mechanical means, and/or any other suitable delivery mechanism), a heating and/or cooling device to deliver a perceivable change in temperature (or heat flux) to the body (or a specific part thereof), and/or generally any means suitable to deliver a signal to the patient that is perceivable by the patient's senses. Optionally, the stimulus source 120 may also comprise one or more devices for directly (but not necessarily invasively) stimulating neural tissue in the patient's brain, e.g. a Transcranial Magnetic Stimulation (TMS) coil (and/or system), a focused ultrasound transducer (and/or system) and/or other such devices for stimulating the brain (preferably in a well-controlled specific region), e.g. such as to enable the analysis of its effect in the directly stimulated brain region and/or brain regions functionally linked thereto.

The stimulus source(s) 120 may be used, in performing a method in accordance with embodiments, to trigger activity in one or more brain regions (and/or brain circuits) of interest in the subject, without necessarily requiring a voluntary (physical or mental) action of the patient in response, even though functional MRI (fMRI) protocols (paradigms) that presume and/or rely on a voluntary response of the patient are not excluded either. The stimulus, such as an auditory or visual instruction, may cue the patient to take a specific mental action in response to the signal. For example, the patient may be requested to indicate his/her understanding of the instruction by attempting to focus thought, by attempting to move a part of the body, to remember specific events (or memories of a predetermined type), and/or to undertake any other mental activity.

The timing of the stimuli may be controlled with respect to the timing of the MRI data collection (and/or vice versa), such that acquired images can be analyzed by taking the stimuli presented at the time of image collection into account (or prior to the time of image collection by an at least approximatively known time delay).

The RF transceiver 116, the gradient coil controller 118 and the stimulus source(s) 120 may be connected to a hardware interface 121 of a computer system 122. The hardware interface may, for example, be a digital network interface or data bus interface. The hardware interface may be adapted for exchanging data and/or commands between the computer system and other components of the system. The hardware interface may also be adapted for exchanging data and/or commands between different components of the computer system. The hardware interface is not necessarily a homogenous interface infrastructure, e.g. may comprise or consist of different types of data exchange media, computer networks, communication buses, shared memory access structures and the like, and/or may be distributed over a plurality of processing devices.

The MRI system 100 is, particularly, adapted for acquiring fMRI images. In other words, a timeseries of images can be acquired, in which each image in the sequence is reconstructed, e.g. by the computer system 122, based on signals detected by the RF transceiver and the antenna coil(s) connected thereto. For fMRI imaging, the RF coil(s) may be, specifically (or, at least, typically), integrated in a head coil assembly to achieve a good signal quality for imaging the patient's brain.

Embodiments in which the computer system comprises a plurality of computers, interconnected by a suitable network infrastructure are not necessarily excluded. For example, processing performed by the computer system may be distributed over a computing cluster or cloud computing platform. Also, while reference is made to a conventional computer architecture hereinbelow, it will be clear to the skilled person that this is not necessarily the case in all embodiments. For example, the computer system may comprise, or even consist of, dedicated hardware for implementing (e.g. some of) the processing functions described hereinbelow, e.g. comprising an application specific integrated circuit and/or field-programmable gate array hardware. It will also be understood that, where reference is made to a single computer system, different functions of this computer system may also be assigned to separate dedicated computer systems, e.g. which are able to exchange data. A distributed computer (and/or other processing and/or control) infrastructure, e.g. for controlling different aspects of the operations, may be adapted to use a central reference clock signal or may use similar synchronization means to enable (different parts of) the system to control and/or account for the relative timing of stimuli, gradient and/or RF signals and data acquisitions.

The computer system 122 may comprise at least one processor 123. The processor may be operably connected to the hardware interface, such as to allow the gradient coils, the sensory stimulus source and the RF transceiver to be controlled by the processor and/or to enable processing of data received by the RF transceiver by the processor.

The computer system 122 may comprise a user interface 124, e.g. for interacting with an operator operating the MRI system. The processor may likewise be connected to the user interface, e.g. such that a user can select actions to perform by the MRI system, parameters to apply, review data indicative of the operation of the system and/or images as acquired by the system (or images or data, e.g. output data, derived therefrom). The user interface may comprise a display, a keyboard, a pointer device, a touch interface, and/or other such components as are ubiquitous in human-computer interaction systems. The user interface may be adapted to render images, e.g. via the display.

The computer system 122 may comprise a memory 125. The memory may comprise or consist of different types of memory, all of which are (directly or indirectly) accessible to the processor. This may for example include a random access memory, a cache memory, non-volatile memory (such as flash memory), hard drives, solid state drives, and/or other storage devices. The memory may comprise a non-transitory computer-readable medium.

The memory may contain machine-executable instructions to enable the processor to perform various data processing tasks and/or to control other components of the MRI system. The machine-executable instructions may enable the processor to perform at least part of a method in accordance with embodiments of the present invention.

A method in accordance with embodiments of the present invention may be implemented by a source program, an executable program (e.g. a bytecode or intermediate language code, an object code and/or a machine code), a computer-interpretable script and/or any other entity comprising a set of instructions that can be directly or indirectly (e.g. by means of facilitating programs or codes, such as interpreters, just-in-time compilers, compilers, etc., and/or supporting code libraries, frameworks, an operating system, etc.) executed by a computer system, e.g. by the processor of the computer system 122. It is pointed out that embodiments are not limited to computer code implementations, e.g. the method may be performed (completely or in part) by dedicated hardware specifically designed for the intended method, e.g. in an ASIC, and/or by a hardware configuration that can be loaded in a programmable hardware construct, e.g. an FPGA.

In further aspects, the present invention relates to a device, e.g. the computer system 122 and/or such specifically adapted and/or programmed processing hardware, adapted to perform a method in accordance with embodiments of the first aspect of the present invention, and to a computer-program product adapted to perform a method in accordance with embodiments of the first aspect of the present invention when executed by a computer.

The memory 125 may also contain a representation of pulse sequence commands, which enable the processor to control the MRI system in a corresponding manner, e.g. by controlling the gradient coils and RF transceiver in accordance with these commands. The memory may also contain magnetic resonance imaging data acquired by controlling the MRI system with the pulse sequence commands.

The memory 125 may comprise a representation of stimulus commands (or data) to control the stimulus source 120. These stimulus commands may be associated with, or integrated in, the pulse sequence commands, such that, upon execution, a well-defined relationship in timing between the operations performed by the gradient coils and RF transceiver (and/or further components of the MRI system) and the stimuli provided to the patient via the stimulus source is established. For example, the stimulus commands may control or induce a change between an active state, in which a specific stimulus is performed, and a control state, e.g. in which an alternative or no stimulus for reference/control is applied, between different acquisitions of the MRI data. Metadata may be associated with the acquired MRI data to indicate a state of the stimulus source(s), e.g. parameters controlling the stimulus source and/or indications of the (type of) stimulus that was presented to the subject, at the time corresponding to the time of acquisition of the MRI data.

The computer system 122 may also be adapted to reconstruct a tomographic image from MRI data acquired from the RF transceiver. Methods for controlling the MRI system using a suitable pulse sequence and for reconstructing tomographic images from the signals collected by the RF transceiver in response are well-known in the art, and will not be discussed in detail in the present disclosure. However, it is noted that the pulse sequence will typically comprise commands or data to control the MRI system such as to collect image data (as reconstructed by corresponding processing of the acquired signals) that is sensitive to changes in the human brain due to brain activity. These changes may rely on a hemodynamic response of the brain in response to brain activity, e.g. an increased blood flow and/or changes in the rate of consumption and/or supply of resources in the blood in active regions of the brain, and/or on a blood-oxygenation level dependent signal contrast.

The computer system 122 may comprise, e.g. may implement via suitable software and/or hardware, an analysis module to analyze (a timeseries of) fMRI images, e.g. taking a schedule of stimuli provided during the acquisition of the fMRI images into account, and to output a result thereof, e.g. as explained in detail hereinbelow. The output may be provided via a user interface, stored on a fixed or removable storage medium, exported via a data communication network interface, and/or in another suitable form to make the result available to a human or machine user, e.g. for review by a clinician, storage and/or further use by a (e.g. automated) processing system.

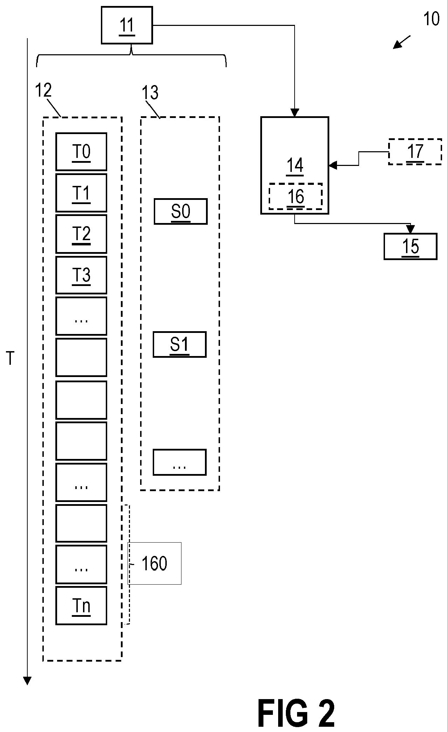

Referring to Fig. 2, an illustrative method 10 for analyzing neural activity in the brain of a patient by functional brain imaging to diagnose, assess, follow-up and/or classify a migraine condition in accordance with embodiments of the present invention is shown. Even though the primary symptoms of migraine may be easily identified, a differentiation from other conditions (e.g. cluster headaches) and/or between different types of migraine, a grading of the intensity of the migraine condition and/or a predictive approach for detecting future attacks and their timing, as can be provided by embodiments of the present invention, could have a substantial impact on the overall quality of life of the patient and/or the efficiency of clinical resource usage. By providing quantitative and objective metrics (e.g. for one or more of the aforementioned applications) and/or related tools, a better (e.g. more effective) use of therapeutical modalities may also be achieved, e.g. by enabling (or facilitating) an accurate (differential) diagnosis and/or an objective patient follow-up procedure.

The method 10 comprises acquiring 11 functional brain imaging data, e.g. one or more images of the patient's brain from which functional information (e.g. as opposed to purely anatomical information, even though such images may typically comprise both anatomical and functional information to some extent) can be deduced (e.g. extracted, inferred, ... by suitable processing). The images may typically be tomographic images, e.g. volumetric (3D) tomographic images. The images may be of a radiological and/or nuclear medicine modality, e.g. magnetic resonance images, single photon emission computed tomography images, positron emission tomography images, and/or other such modalities (insofar functional information can be deduced therefrom), but, by extension/generalization, this may also comprise spatially (and possibly also temporally) resolved information from which neural physiological functionality can be deduced, e.g. electro-encephalography (EEG), magnetoencephalography (MEG) and possibly other such modalities.

Acquiring 11 the functional brain imaging data may comprise acquiring 12 a timeseries of functional magnetic resonance images of the brain of the patient using a magnetic resonance imaging system 100, e.g. an MRI system 100 as discussed hereinabove. The vertical axis T in Fig. 2 illustrates a flow of time, e.g. in the acquisition of the sequence (timeseries) of images. Acquiring the timeseries of images may comprise controlling the MRI system in accordance with a pulse sequence, and collecting signals by the RF transceiver. The timeseries thus comprises a sequence of images acquired during subsequent time blocks T0, T1, ..., TN (or substantially acquired at a sequence of instants in time T0,T1, ..., TN).

Acquiring the timeseries may also comprise processing the collected signals to reconstruct an image, e.g. a tomographic image. The reconstructed image may be a volumetric (3D) tomographic image, one or more planar (cross-sectional; 2D) images, or a combination thereof. The reconstruction may, for example, be performed after acquisition of the timeseries, or may be performed during the acquisition, e.g. updating results while the examination is still in progress based on the data acquired up to that point in time. The acquired signals may be representative of substantially a same instant in time for different locations in the brain (e.g. using suitable 3D imaging sequences and/or accelerated imaging techniques), or may be representative of slightly different instants in time for different locations in the brain (e.g. using a conventional echo planar imaging technique), which may be corrected (to a reference point of time for the set of different slices) as known in the art (e.g. using a slice timing correction method). Therefore, the (if needed, processed) timeseries comprises images (2D or preferably 3D) that are substantially (or at least approximatively) representative for the imaged brain at corresponding points in time. Suitable MRI and fMRI techniques are well-known in the art, and therefore not discussed in detail, e.g. suitable pulse sequences, image reconstruction methods and/or fMRI analysis techniques (e.g. to create images representative of the brain activity, e.g. statistical maps of assumed brain activity) as known in the art can be applied. Typically, the BOLD effect is used in such techniques as proxy for neural activity in order to localize activity in the brain, e.g. by estimation from MRI images that have a suitable sensitivity to blood oxygenation dependent effects, e.g. T2^{∗}-weighted images. As an example, the acquired fMRI data of the timeseries may comprise a plurality of MRI images at a plurality of different echo times following an application of an excitation RF pulse, e.g. such that each image corresponds to a time sample point in the timeseries.

Acquiring 11 the functional brain imaging data may also comprise providing 13 a plurality of stimuli S0, S1, ..., at different points in time, e.g. via one or more stimulus sources 120, to the patient during the acquisition of said fMRI timeseries to induce activity in the patient's brain. The stimuli, e.g. sensory stimuli, may comprise displaying a visual cue, emitting an auditory cue, providing a touch sensation, a heat (and/or cold) sensation, a pain sensation, a smell, a taste and/or other such types of perceptual stimulus. The stimuli may also (additionally or alternatively) comprise the stimulation of brain tissue by direct or indirect manipulation, e.g. by transcranial magnetic stimulation (TMS), deep brain stimulation (DBS) and/or high-intensity focused ultrasound (HIFU). The stimulus (or different stimuli of generally the same type) may be repeatedly provided, so as to accumulate sufficient statistical power to deduce their effect in the brain from the acquired images. Different sets of different stimulus modalities are also not necessarily excluded, e.g. such that their respective effect(s) in the brain can be compared (e.g. contrasted) to each other. Thus, in embodiments, stimulation of the brain by presenting sensory cues, such as video, audio, pictures and/or tactile/haptic signals, and/or olfactory or other sensory signals, can be used to determine their effect in the brain of the patient, as can be deduced (by suitable processing) from the acquired images.

Thus, external signals may be applied to the patient to evoke a specific response in the patient's brain, particularly during the functional brain imaging examination (e.g. a fMRI, PET, ... session). The stimuli may be typically applied to induce an effect in the brain so as to observe changes in the brain indicative of this effect, e.g. to localize brain tissue directly or indirectily involved in this effect (or modulated by this effect) in space and/or time. In a more generalized sense, the stimulus and its effect of interest may also relate to a mental action, memory, thought, feeling and/or other internal state of the brain that is generated by the patient at a specific time or in a specific time window, e.g. in response to an instruction, cue and/or guideline provided in accordance with the functional imaging protocol. Stimuli may also (additionally and/or alternatively) be delivered directly or indirectly to the patient's brain, e.g. via an electromagnetic and/or mechanical signal, such as by transcranial magnetic stimulation and/or ultrasound stimulation of brain tissue.

The effect, e.g. the brain response, is typically localized in time, e.g. occurs at the moment when the stimulus in provided or shortly thereafter, e.g. on a scale of a second up to minutes after the stimulus (or a sequence of similar stimuli). Thus, the (direct) stimulus response has a specific temporal relationship to the time of providing the stimulus. The response of interest is, in a sense, also localized in space, e.g. occurs in a specific brain region or regions, along predetermined neural pathways, or can similarly be identified in the spatiotemporal fMRI data (and/or by other suitable imaging modalities, e.g. PET data, MEG data, ...). Thus, the effect of the external stimulus and/or of the induced internal state in the brain can be observed and detected at the time or in the time window where it is presumed to be detectable in view of the timing of the stimulus (cue, instruction, ...) by analysis of the acquired images.

For example, the stimulus may be a sensory stimulus, e.g. which may evoke a response in brain regions linked via one or more sensory organs and neural pathways, possibly up to higher level functions, such as conscious thought, emotional response and/or memory recall, depending on the specific design of the stimulus. The neural response of interest may involve an emotional response, a mood shift, a sentiment, a memory, conscious thought or the like, and/or lower level functions, such as autonomous, subconscious and/or involuntary activity in the brain, e.g. activity of brain areas providing the direct mechanics of visual, auditory or other sensory reception.

The effect in the brain that is intended to be observed after processing of the (fMRI, PET, MEG, ...) functional images is not necessarily the direct effect of the (e.g. sensory) stimulation, but may rather target (or target as well) an indirect effect of interest. For example, even though stimuli are provided in the form of visual content, e.g. images, visible text, motion picture clips, ..., the stimuli may be designed to evoke a memory effect, test a higher level mental function (e.g. cognitive functions), test emotional processing (e.g. emotional arousal, valence, ...), and/or test the shifting of attention from and to the displayed cues. Thus, the expected indirect effects of the stimulation may be used to derive a quantitative measure (or measures) of the activity in those brain regions that are involved in the higher-level processes (e.g. for which the stimuli may be specifically designed).

Whereas the present detailed description focuses primarily on embodiments using functional magnetic resonance imaging, the skilled person will understand that the same principles can be easily applied to other imaging modalities. For example, positron emission tomography images may be collected for at least two different states, e.g. while applying stimuli as discussed hereinabove and in the absence of such stimuli (or using different stimuli that are designed to result in a suitable control state), in a manner that is, except for some practical implementation differences as well-known in the field, substantially analogous.

Specifically, in embodiments of the present invention, the stimuli may comprise (without limitation thereto) nociceptive stimulation (e.g. using a noxious chemical, e.g. ammonia gas, and/or thermal pain stimulation) to observe an effect on the brain's processing of pain signals and/or higher-level processes, and/or to detect atypical and/or dysfunctional brain regions and/or brain region interactions of the patient vs. healthy subjects and/or between different types of migraine. For example, generally, an imbalance in activation patterns between pain-facilitating regions (higher activity vs. healthy subjects) and pain-inhibiting regions (lower activity vs. healthy subjects) may be observed, and thus used for diagnostic and/or prognostic purposes.

Embodiments of the present invention may also (in combination therewith and/or in isolation and/or alternatively) use visual stimuli, e.g. to detect atypical regional activity and/or interaction of brain regions (vs. e.g. healthy subjects and/or between different classes of migraine). This approach has the advantage of potentially being less unpleasant to the patient than nociceptive stimulation, and/or may advantageously use a link of migraine with hypersensitivity often observed in migraine patients.

Embodiments of the present invention may also use (additionally and/or alternatively) stimuli in accordance with an attentional processing task, e.g. a Posner cueing task (and/or a variation thereof as known in the field). The latter Posner paradigm (without limitation thereto) has been extensively used in other applications to assess disorders and study the brain's spatial attentional processes, and may be particularly useful in view of the association of migraine with hypersensitivity and a possible mechanism of dysfunctional suppression of non-attended events.

While imaging during a migraine attack (ictal) may be useful to gather functional imaging data to characterize the migraine condition and/or derive useful clinical indicators therefrom, it will be understood that, for practical purposes, the patient is preferably imaged in between attacks (interictal). However, embodiments are not necessarily limited thereto. For example, an attack may be actively triggered under controlled conditions to gather imaging data, and/or predictive measures (e.g. as discussed in the present application) may be used to plan an imaging session when an attack is imminent. Nonetheless, brain activation (and/or regional brain activity correlations and/or additional imaging information, such as morphometric characterization of brain regions) indicative of migraine and its associated dysfunctional processes in the brain that are observable under 'normal' (e.g. pain-free, or at least attack-free) conditions may be primarily of interest, e.g. preferable due to the impracticality of planning an imaging session on short notice and/or at an only approximatively defined time, and/or in view of potential concerns due to possibly aggravating (long-term) effects of induced attacks.

Whereas the discussion hereinbelow may primarily apply to analysis of functional images acquired in the interictal phase (between attacks), a same or similar analysis may be used for images acquired in the ictal phase. However, during an attack (or closely before/after), specific further activation patterns may be exploited to gain insight. For example, migraine patients may show a substantial increase in brain activation in the prefrontal cortex and/or the temporal lobe during an attack (vs. normal and/or vs. other types of headache), e.g. as measured by PET and/or fMRI. For example, without limitation thereto, the medial temporal lobe may show increased activity and/or increased functional connectivity to other brain regions (e.g. which may be detectable in the ictal phase as well as in the interictal phase, even though the magnitude of activation may differ, e.g. be stronger in the ictal phase). It will also be understood that stronger activation may be detected (e.g. vs. interictal) in regions linked to pain processing, e.g. as discussed hereinbelow in detail, e.g. in the brainstem, thalamus, insula, cingulate cortex, ..., and/or linked to the sensorimotor network, e.g. cerebellum, pre/post central gyrus, temporal lobe, ... As a further example, during attacks, mu-opioid binding may be examined by PET imaging to detect reduced (vs. normal subjects and/or vs. the interictal state of the patient) mu-opioid non-displaceable binding potential In e.g. the cingulate cortex, the nucleus accumbens, the thalamus, the periaqueductal gray and/or the medial prefrontal cortex.

The plurality of stimuli, i.e. each stimulus or subset of stimuli (e.g. when multiple different sets of stimuli are used, for example to test their differential brain responses, and/or different combinations of such stimuli are used) may thus be provided 13 during the acquisition 11 (e.g. while acquiring 12 an fMRI timeseries). For example, at least one image (e.g. of the timeseries) may be acquired while an effect of the stimulus in the brain is presumed to be present. In addition to this at least one image acquired (e.g. shortly) after or during the stimulus application, at least one other image (e.g. of the timeseries) may be acquired while the effect of the stimulus can be assumed to be absent (or vice versa, and/or to a lesser or greater extent), e.g. preceding the stimulus or a sufficiently long time after the stimulus. As generally known in the art, the effect of the stimulus can be evaluated by comparing these images. It is to be noted that, typically, a relatively large number of images may be acquired and processed to determine the activity of interest in the brain accurately (e.g. particularly when using fMRI, which may have a relatively low signal to noise ratio for the effect/effects to be detected), e.g. at least ten images in the absence of the effect of interest and at least ten images in the presence of the effect (without implying any limitation by this example). It will be understood that absence and presence may refer to greater and lesser activation and/or deactivation, and not necessarily to a strictly Boolean condition, and/or that a contrast of interest may compare two or more conditions in which stimuli are used that are different in a predetermined manner (e.g. a Posner task) to show a relative difference in their associated activation patterns. Thus, the (type of) stimulus may be typically repeated, such as to repeatedly induce the brain activity of interest and/or to increase sensitivity by joint analysis. In other words, multiple stimuli corresponding to the same task paradigm condition may be provided, e.g. to obtain sufficient detection power in assessing this stimulation class/setting, while, across different stimuli (sets of stimuli), the type, combination, content class, one or more relevant parameters, ... of the stimuli may also be varied to study the effect(s) for these conditions, e.g. to allow the effect of different sets of stimuli to be compared relative to each other.

The stimulus (or stimuli of a same type, nature, content and/or corresponding to a same test paradigm condition) may be repeated during a first block of time, while it may be absent during a second (e.g. next) block of time, in which these types of block may alternate for a number of times, e.g. to increase robustness of the test performed. Different types of stimulus, e.g. differing by type of content, modality, sense (sensory modality) and/or parameters of the delivery, may also be studied. In a blocked design, different types of stimuli, e.g. painful heat application, noxious chemical exposure, visual stimuli, attentional cues, ..., may be delivered in different blocks, e.g. each block may be attributed to a specific stimulus condition (optionally in addition to a block or blocks of a control condition without stimuli or with neutral stimuli). Thus, the simple 'alternating' block design example can readily be extended by creating a sequence of different (types of) blocks (potentially including one or more control condition blocks). The order of the types of blocks may be randomized, e.g. in a balanced design to avoid crossover effects. Such blocked-trial designs may advantageously provide a good sensitivity, e.g. a significant detectable signal (or, particularly, a signal change due to the applied stimuli) in image voxels or regions of multiple voxels from which the response to the stimulus in the brain can be determined. Furthermore, a blocked-trial design may advantageously reduce the dependency of results determined from the (e.g. fMRI) data, e.g. by statistical analysis, on timing-specific (or strongly timing-sensitive) parameters that may be a-priori unknown or only approximately known, such as a hemodynamic response function (e.g. latency thereof), the fluency or ease of neural signal transmission along neural pathways and/or the activation flow coupling response. Thus, a blocked-trial design may be an advantageously simple and robust approach to gather the data of interest.

Alternatively to, or in combination with (in a hybrid paradigm), a blocked design paradigm, i.e. an "event-related" (e.g. fMRI) approach, may be applied. In such approach, the stimuli are considered as separate discrete events (points in time), such that neural processes of interest, e.g. pain sensation, attentional shifts, etc., e.g. the brain response associated with each stimulus event, can be analyzed independently and/or by a model that does not necessarily rely on a straightforward averaging of the effect within a series of consecutive stimuli (i.e. in a single block) or a conventional mathematical extension of such averaging (e.g. fitting a block function or convolution of a block function with a response primitive, e.g. a hemodynamic response function, HRF). This may advantageously allow a more accurate (analytical) separation of, for example, a low-level sensory effect of the stimulus (e.g. initial effects which generally do not involve consciousness or awareness as such) and a (e.g. cascade of) higher-level brain responses, such as triggered memories, emotional responses, (e.g. pain) sensation associated with a triggering sensory event, thought processes, attentional shifts, etc. Even though explicit control events may be included, e.g. a similar stimulus to the test stimulus, in which this similar stimulus is presumed to have no significant (or a substantially different) effect on the process(es) of interest, it is an advantage of an event-related protocol that the isolated trials can be immersed in plenty of non-trial data sampling (time) points, e.g. such that sufficient data representative of the control condition is readily available. A more intricate analysis (e.g. as opposed to a simple binary analysis of control/effect, which e.g. may be applied to a block paradigm, without limitation thereto) furthermore allows to quantify an effect of the stimuli that occurs over time, e.g. using a declining (linear, exponential, ...) decay model (or e.g. a delayed peak model after the event) of the sought effect after each stimulus of interest, e.g. such that each time point may be considered as a (non-binary) mix of the "control" and "event" state (or, by extension, possibly including multiple different event states).

It will also be understood that one or more parameters of the stimuli provided may be taken into account in the analysis of the functional imaging data, e.g. a pain stimulus intensity may be varied over different stimuli (e.g. across trial events or blocks) and this associated parameter value (e.g. non-Boolean and/or real-valued; but discrete, categorical, ordinal and/or Boolean parameters may obviously also be studied) may be taken into account in the analysis (e.g. as a covariate).

In embodiments in accordance with the present invention, the stimuli that are provided may comprise a plurality of types of sensory stimulus, e.g. using different sensory modalities (visual, auditory, touch, heat, smell, ...) and/or different content type (e.g. visual stimuli of a different nature, e.g. light flashes, images with a specific content, ...) and/or different parameters (e.g. different frequencies of stimulation, different requirement of attentional and/or cognitive involvement of the patient, ...). These different types of stimulation may be analyzed as different conditions, e.g. such as to compare and/or contrast their effect on the brain, and/or may be taking into account in the analysis as covariate or other model parameters (e.g. a parameter of the stimulation that is varied over a range may be included as a model parameter in the analysis). This allows to specifically study which type of sensory stimulation (or their specific characteristics) may be responsible for triggering a migraine attack (without limitation) for a specific patient. Thus, information gathered and analyzed in accordance with embodiments may be used to calibrate a patient's migraine response and/or in an individualized migraine prevention strategy. When the patient is exposed to sensory triggers and stressors (which may be tailored to the specific patient, e.g. by taking accounts of the personal experiences of the patient into account) during the functional imaging, the functional imaging data thus collected may be used in determining the output(s) of the method to gain insight in the specific type of migraine (e.g. classifying the migraine case as function of its specific attack triggers) and/or improve and/or optimize a treatment and/or prevention plan. For example, such treatment/prevention plan may include (e.g. lifestyle) guidelines, advice and/or medication optimized for the patient. The study of the effect of different stimuli may also be combined with other (e.g. fMRI) task/test paradigms as discussed in the present disclosure, e.g. cognitive tests, attentional processing tests (e.g. a Posner task) and/or pain response tests.

The method 10 for analyzing neural activity in the patient's brain furthermore comprises determining 14 at least one activity measure indicative of brain activity in at least one predetermined region of interest in the patient's brain based on said functional imaging data, e.g. the timeseries of fMRI images. The brain activity may express the brain activity in response to the provided stimuli, e.g. relative to (activity in said brain region in) a control state (e.g. the absence of stimuli, under application of a different type of stimuli, in a different combination of applied stimuli, and/or another such relevant control condition). Additionally or alternatively, the brain activity may indicate a relationship between activity in one or more different brain regions, e.g. a correlation (but not necessarily a simple linear correlation, e.g. other corelating measures, such as mutual information, are not necessarily excluded) of activity within a brain region and/or in a brain region relative to another brain region.

For example, a resting-state functional connectivity magnetic resonance imaging (rs-fcMRI) analysis may be performed 16 (possibly in combination with a conventional stimulation-based fMRI protocol, e.g. a blocked and/or event-related fMRI paradigm, as discussed hereinabove) to determine the functional connectivity of different brain regions (also discussed in more detail hereinbelow). In other words, acquiring 11 the functional imaging data may comprise acquiring 160 resting-state functional imaging information, e.g. indicative of brain activity without externally applied stimulation, and processing 16 the acquired functional imaging data to determine resting-state functional connectivity of brain regions of interest.

Whereas the present disclosure discusses data acquisition by fMRI imaging in detail, it will be understood that, alternatively, other modalities may be used, such as PET. Furthermore, further imaging data may be acquired (of the same patient) to complement the gathered functional (e.g. fMRI) imaging data, and/or other data may be additionally used, such as physiological and/or other relevant data of the patient, e.g. an electroencephalogram (EEG) or information derived therefrom, blood pressure and/or other cardiovascular parameters, current and/or past drug use and/or other information from the patient's medical history, and/or other such side-information. This further data can be taken into account in determining the activity measure, e.g. by using it as additional input, i.e. combined with the functional imaging (e.g. fMRI) data, and/or may be combined with the activity measure(s) in determining the output (e.g. a migraine classification, migraine diagnosis score, proposed therapy, ...). For example, the functional imaging paradigm(s) (e.g. the sequence and nature of provided stimuli) may (depending on the task paradigm) be adapted to provide such additional data (e.g. data concomitantly obtained with the functional image acquisition), e.g. measurements of response times to cues, eye tracking information, pain trigger response measurements (e.g. heartrate, skin resistance, ...), such that this additional data may be readily taken into account in processing the functional images, as known in the art (e.g. as covariates in a GLM or similar processing technique, without limitation thereto).

Additionally, it will be understood that the functional imaging data as such may also comprise different modalities. For example, PET, MEG and/or EEG data may be acquired concomitantly with fMRI data, such that relevant brain activity (e.g. associated with the applied stimuli, without limitation thereto) can also be detected from this additional source of information, e.g. to improve sensitivity and/or robustness.

Thus, the method may comprise receiving 17, as further input, further imaging, physiological, medical, behavioral and/or diagnostic data and taking this further data into account in determining 14 the activity measure and/or an output value based on said activity measure, e.g. a migraine (diagnostic) scoring or staging value (e.g. as a personal migraine score index), a migraine classification, a predictive value (e.g. of migraine frequency and/or time to next attack), etc. Such further data may include clinical input, such as (codified) medical history information, physiological data (heart rate, heart rate variability, stress level measurements, respiratory rate, etc., e.g. which may be collected by a wearable device for continuous and/or long-term monitoring), information pertaining to the patient's schedule, lifestyle events and/or notable sensory triggers, and/or more subjective information, such as self-reported information of the patient's experience before, during and/or after attacks (which, e.g., may be collected by a portable device, such as by a smartphone app).

Determining 14 the activity measure(s) may comprise comparing at least one first image (e.g. of the fMRI) timeseries acquired while an effect of the stimulus in the brain is presumed to be absent and at least one second image (e.g. of the same fMRI timeseries) acquired while a direct or indirect effect of the stimulus in the brain is presumed to be present (or, more generally, the effects are presumed to differ substantially in the brain region or regions of interest between the first and second image/images). For example, determining the activity measure(s) may comprise determining a relative change in activity compared to a control (or different) condition in the acquired fMRI data, for at least one region of interest in the brain that is linked to pain signal processing and sensation, attentional processing, executive function and/or sensory processing (e.g. vision). This list is not necessarily exhaustive. For example, brain regions involved in mind-wandering (resting wakeful state maintenance, e.g. which may be analyzed by rs-fcMRI processing) may also be of particular interest, and/or, for example, memory function, emotional processing, auditory processing, sensorimotoric function and/or another higher-level brain function.

For example, a first image, e.g. an fMRI image of an fMRI sequence (timeseries), may be acquired before a stimulus was applied, and a second image may be acquired during or (e.g. shortly) after providing the stimulus, e.g. less than one minute after, e.g. preferably less than 30 seconds after, e.g. less than 10 seconds after. Comparing these two images allows activity in the brain to be detected and determined that is evoked by that specific type of stimulus. Comparing at least the first and second image may be interpreted broadly, e.g. may involve any operation that involves image information from the first image and the second image to determine a qualitative or quantitative measure that indicates brain activity that is substantially different between the two conditions. This may involve the calculation of an image subtraction, an image division or similar measure of contrast. In view of the subtle contrast differences observed in fMRI, e.g. due to the blood oxygenation level dependent (BOLD) effect, it will be understood that typically a first set of images is compared to a second set of images, each corresponding to a substantial number of time points, to be able to detect the relevant contrast changes indicative of brain activity. It will also be understood that this comparison to detect contrast changes may involve a more complex model, e.g. by fitting a general linear model, generalized linear model or similar statistical model to the data, in which in addition to the indicator variables and/or parameters representative of the 'state' of a time point (e.g. in the simplest case being a binary value to distinguish the time points at which the stimuli are assumed to have effect if detectable from the time points representative of a control condition), a plurality of different parameters may be added to the model, e.g. representative of residual changes due to motion, time-dependent changes in the effect(s), constraints and/or other such variables. A predictive model of the expected brain activity may be built by taking time-dependent effects of the stimuli into account, e.g. assuming that at the time of stimulation, the effect is not yet detectable, and assigning to following images a weight based on a model of the signal response, e.g. the hemodynamical response function (HRF). Many variations are known in the art to specifically model this response. For example, the approach for a block-based design may resemble fitting a wide flat curve that is slightly offset (e.g. delayed) in time with respect to the timing of the 'active' block of stimulation (e.g. obtainable by convolution of the HRF with a boxcar function representing the timing of the block), while the approach for an event-based design may resemble fitting the data to a sequence of less easily identifiable values (e.g. each representing the sum of HRF convolutions with delta functions for indicating the preceding relevant stimuli at the time of image acquisition for which that value is determined).

As is generally well-known in the field, each voxel of the image may be fitted to such model to obtain an estimate of neural activity linked to the stimuli (or for each type of stimulus) for that point in the brain indicative of.

This may also involve suitable pre-processing and/or processing steps, e.g. to compensate for motion between the images, to correct timing, to register the images to a known coordinate framework, e.g. such that the region of interest can be easily found in the images (and the time evolution of the detected single for each point in the brain of interest can be easily retrieved and analyzed), image smoothing, and such. For example, image smoothing may be applied to allow statistically robust inference (for example, based on Gaussian Random Field properties), and/or slice timing correction may be applied to correct for small differences in the actual (center) time of acquiring slices (or other subsets) for a same image of the timeseries.

A general linear (or generalized linear or other type of) model may assign a strength and/or confidence to the brain activity in each voxel explained by the (e.g. separately for each, and/or in combinations and/or contrasts thereof) stimuli condition(s) (e.g. in contrast to a control condition with different and/or absent stimuli) and/or for each region of interest (e.g. which may comprise multiple voxels, e.g. grouped based on a brain region map, a brain region segmentation and/or similar anatomical/topological information). Alternatively (or in combination), a machine learning algorithm may be trained to recognize relevant brain activity based on the input images (or an input vector, e.g. of image and/or timeseries features, derived therefrom).

It is noted that such approach is not necessarily limited to fMRI image processing. For example, except for e.g. possibly straightforward differences in pre-processing steps (as known in the art), a GLM may also be applied to a timeseries of PET images, and/or to a spatiotemporal representation (image timeseries) of MEG data. Images of different modalities (e.g. PET, fMRI) may be combined in a suitable (e.g. multi-level) GLM approach. Such data (and/or other relevant data, e.g. EEG data) may also be taken into account as covariate data and/or confounding parameters in the (primary) analysis of (e.g.) the fMRI data, e.g. by inserting the data (and/or a vector or vectors derived therefrom) in the design matrix of a GLM and/or by adjusting weights of time points and/or spatial regions in the fMRI data (e.g. given a higher weight to time points and/or regions of the fMRI data when/where corresponding activity is suggesting by PET, EEG or other side-information data). In another approach, fMRI data and further data, e.g. PET data, MEG data, EEG analysis data, ..., may be analyzed independently, and their results may be combined to determine a combined activity measure (in a following analysis step and/or combining calculation).

Determining 14 the activity measure(s) may comprise applying a trained machine learning model to the fMRI (and/or other modality) images (and/or derivative information therefrom, e.g. using a low-level salient, e.g. primitive, feature extraction applied to the images as input) to receive the activity measure(s) as output. Thus, the analysis may be performed, e.g. using a processing unit or computer, by artificial intelligence. It is an advantage that a machine learning model can be trained to detect the brain activation/deactivation patterns in relevant brain regions and/or correlations between relevant brain regions. This may avoid difficulties and challenges in designing and finetuning a handcrafted algorithm to obtain the same result, and artificial intelligence techniques have been shown to often outperform conventional algorithms in sensitivity, which may be a key factor in view of, for example, the typically noisy fMRI data from which statistically significant effects are often difficult to detect with a high degree of certainty.

It is also an advantage that a machine learning model can be trained on outputs that are not directly or easily determined by human observation or interpretation of the data. For example, a training set of data may be used that follows up on patients over a prolonged period of time after collecting the imaging data, such that actual clinical outcomes, e.g. the observed response to applied treatment strategies, are known and available as training output values. Thus, actual (retrospective) prognosis data of the patients can be used to train a model to determine such prognosis from the initially acquired fMRI scans. As an example, a conventional trial-by-error strategy may be followed, in which a patient is assigned to a therapy program based on chance and/or the experience and judgment of an attending doctor, with the difference that at least one imaging session is performed to acquire the functional imaging data (or repeated imaging sessions to implicitly provide information on the evolution of the patient), e.g. in accordance with a suitable fMRI paradigm format of stimulation (and/or resting state functional imaging).

The patient may then be followed up by standard checkup procedures, and the progression of the patient may be quantified based on observable changes over a longer timeframe, e.g. using self-reporting information, migraine attack frequency tracking, subjective pain intensity evaluations, drug (and/or other therapy) effectiveness analysis, drug side-effect observations, etc. Once this follow-up data is available, the fMRI data and clinical outcome can be used as input and output (as one training sample, obviously repeated for a number of patients) to train the machine learning algorithm. The machine learning algorithm may thus be able to detect indicative brain patterns in the imaging data (e.g. fMRI scans), correlate this automatically with improved outcomes later and, given sufficient data, filter out ineffective and/or suboptimal therapies (e.g. by the training algorithm reducing the weight of any brain pattern that does not lead to promising improvements in the patient's condition). This approach is obviously not limited to only prognosis and/or therapy suggestion, and can, for example, also be used to classify the type of migraine (after this is established by more conventional methods as training output) and/or diagnose the migraine condition as such, e.g. relative to other, similar conditions (e.g. cluster headaches).

For the sake of clarity, while a machine learning model, e.g. with an output trained to produce a clinical relevant measure (e.g. a diagnosis score, a drug ranking, a migraine classification score and/or other such measure), may not use in a directly obvious way an activity measure of localized brain activity and/or intra/inter-regional activity patterns, it will be understood that such measure indicative of brain activity (e.g. possibly embedded in a trained artificial neural network configuration) may still be implicitly used by the model (given the input it receives and the output it produces), and may therefore be considered to be equivalent or analogous to a more classical (e.g. purely algorithmic, e.g. statistical analysis) approach.

It is also an advantage of machine-learning approaches that side-information, such as PET data, EEG data, self-reporting diary information and/or other such forms of relevant supporting information), can be easily inserted into the model as additional input(s) (raw and/or after suitable processing), e.g. such that the model can automatically determine the relevancy thereof and take this information appropriately into account in producing its output.

Thus, it is clear that an AI-based approach can have many advantages, as discussed hereinabove. However, a more conventional approach (e.g. based on statistical maps, linear models and the like) may have the advantage of better explainability, e.g. can be more readily understood, evaluated and interpreted by humans. Even though significant improvements have been made in the field to create machine learning models that offer better insights in the implicit decision making process applied by the trained algorithm, e.g. to reduce the black-box effect of machine learning, a human-designed algorithm may often still be considered as being better documented and interpretable, with potential implications for quality assurance, accountability and/or due diligence.

In summary, stimuli may be provided 13 at different times during the image acquisition sequence, and/or in different combinations at different times during the acquisition. A model may be used to analyze the produced functional (e.g. fMRI) images. The model may use a conventional, e.g. GLM, approach and/or a trained machine learning model. For example, the stimuli (and/or blocks of stimuli) may be codified in a design matrix of a GLM model, e.g. to provide a suitable representation of the mixing and/or timing of the stimulus conditions as they apply to each acquisition (time sample). The activity measure(s) may be determined 14 by detecting a change in the acquired images of the timeseries in the at least predetermined region as dependent on the different times at which the stimuli are provided.

Determining 14 the activity measure(s) indicative of brain activity in predetermined regions of interest may also comprise, additionally or alternatively, a brain network activation analysis (e.g. of the resting-state or default-mode network), e.g. in which estimated activity (e.g. BOLD values) of different regions of interest (and/or between sub-regions and/or between voxels within a same region) are considered in relation to each other. Thus, the measure of brain activity (or a component thereof) may also indicate a relation between the activity in different regions (and/or within regions), instead of a mere indication of activity in a single corresponding region (or a list of activities in different regions) in response to an external stimulus. Thus, the activity measure(s) for the at least one predetermined region may also consist of or comprise a measure of causal or correlated activation (and/or deactivation with respect to a reference control state) of a plurality of different regions, e.g. to quantify a response of different brain regions working together in response to a stimulation and/or in intrinsic (unstimulated) processes. For example, network analysis and/or resting-state functional magnetic resonance imaging (rs-fcMRI) techniques may be applied to map functional connections, e.g. inferred from correlations between time series corresponding to different regions. This may be combined with (or used alternatively to) a more conventional fMRI analysis to detect changes in brain activity within each specific region of interest in response to stimuli.

The network activation analysis may comprise the calculation of one or more measures of connectivity and/or coherence that express the joint (coherent) activity of different predetermined regions (or activity within such region/regions) in the brain. For example, functional connectivity measures may be determined (without limitation thereto) on the basis of a graph analysis, in which nodes of the graph represent different brain regions and the edges of the graph represent the connections between these regions). Simpler (exemplary) approaches may be based on measures of correlation or association between timeseries representative of different brain regions, e.g. may be based on a covariance matrix calculated for a matrix X = (X₁,X₂,...,Xₙ)^{T}, where each random variable Xᵢ represents a timeseries of detected activity in a corresponding brain region, e.g. by averaging the acquired fMRI signals over the voxels associated with the brain region.

Furthermore, the method may comprise obtaining diffusion-weighted imaging based tractography data (dMRI) to map structural connections in the patient's brain. Such data may be acquired in the same session, or in a previous session (e.g. in view of possibly computationally demanding processing to map structural connections). Thus, an individualized brain connectome may be constructed to identify target regions in the patient's brain to analyze by fMRI (and/or PET, ...) that are relevant for the brain functionality as discussed hereinbelow. For example, a reference (e.g. population-average) normative connectome (e.g. a standardized "wiring diagram" of the brain) may be transformed by the patient-specific dMRI data to remap target areas in the brain to potentially altered locations. Since a reference normative connectome can be obtained at high resolution from public sources, it may be substantially easier to transform (e.g. deform) the reference connectome to fit the acquired patient data, than to produce a patient-specific connectome map from scratch.

The level of detail of the connectome (patient-specific and/or normative) may be adapted for the specific application, e.g. brain areas may need only be defined up to a level that is useful to identify the areas of interest in the patient's brain. In other words, higher resolution connectome data (which may be more difficult to accurately obtain for a single patient) is possibly not required for this approach.

While such additional tractography data may be useful to determine regions of interest for activity and/or connectivity analysis, it will be understood that it may also be used directly (as such or additionally) in determining the output metric(s) of interest, e.g. quantifying abnormalities in the brain's connectome (vs. a normative map) as indicative of the migraine condition (causative, and/or induced by brain damage due to migraine attacks).

It is also to be noted that the functional imaging may also be complented with information obtained from conventional (i.e. anatomical) imaging, e.g. MRI imaging (with typically higher resolution and contrast characteristics optimized for anatomical imaging of the brain, as opposed to e.g. optimized for detecting the subtle BOLD effect in functional imaging). One potential use of conventional imaging in the context of embodiments of the present application that is particularly noteworthy is the analysis of brain morphology based on the acquired images, so as to assess morphologic differences of the patient with respect to references. In view of natural variation in brain morphology (that is, even in the absence of pathologic conditions), Even though such measures might be difficult to establish with high accuracy for a single specific patient (in view of natural variation in healthy subjects as well as migraine patients), deviations of the brain morphology may be quantified and used as additional input in the analysis of the fMRI (and/or PET, ...) data and/or in calculating output metrics derived therefrom.

For example, in calculating the output (e.g a classification, a migraine diagnosis score, ..), the morphologic information may be included with a weighting factor relative to information obtained from the processing of the functional imaging information. As another example (not necessarily mutually exclusive), if a brain region shows a large deviation relative to a reference (e.g. a high absolute Z score when compared to a reference mean and standard deviation), (e.g. fMRI) activity in this region may be given a larger weight in determining an output score (e.g. a migraine probability, a classification vector, ...) than another region that has morphologic properties closer to the standard (i.e. is more in line with the reference). Thus, such information may be taken into account even if morphometric abnormalities for a single patient, when analyzed in isolation, might not reach a level of statistical significance (e.g. the Z score in this example having an elevated magnitude but not necessarily significantly so). Other approaches than weighting a region in the fMRI analysis based on morphometry and/or including morphometric values as additional (e.g. properly weighted/scaled) elements in an output metric calculation may be considered as well for taking the values indicative of morphometric (or morphologic) properties of (a) region(s) of interest into account. Morphometric/morphologic properties may include, for example, a size, density, shape metric and/or other characteristic(s) of the region of interest compared to a reference standard therefor. The reference standard may refer to the distribution of the same property (and same region) in, e.g., a population of healthy subjects (possibly a suitable healthy sub-population with comparable demographics as the patient under study, e.g. gender, age, weight, bone mass percentage, ...), for example so as to compare the observed characteristic(s) to the standard reference by normalization, a Z-score and/or other such comparative measure.

It will be understood that such strategy of comparing and/or normalizing the morphometric/morphologic characteristic to a healthy reference may also be applied to (an) activity measure(s) for a brain region as discussed hereinabove, e.g. to compare the observed activity in a specific brain region to the known distribution of that brain region in a healthy population (and under the same conditions, e.g. fMRI test paradigm), e.g. so as to objectify the observed value(s) in terms of the normal expected distribution thereof in the absence of migraine. The latter is not strictly necessary, but may be useful (without limitation thereto) in defining weights of combining different (thusly reference-population-normalized) activity measures for different brain regions into a single (or few) output values, e.g. a migraine/non-migraine and/or migrain-with-aura/migraine-without-aura classifier, a prognosis or other predictor value, a migraine strength rating, ... It will also be understood that the reference population may also refer to a different type of population, e.g. a population of cluster headache patients, a specific set of migraine patients (e.g. only migraine-with-aura, or without-aura), and/or that this notion may easily be extended in a straightforward manner, e.g. relating a patient's (hypothesized) population to another (null-hypothesis) population, e.g. in a t-test or F-test statistic.

Reference is made to the regions noted hereinbelow, which, even though mentioned in relation to detectable abnormal activity and/or functional connectivity, may also show morphological changes that can be quantified and used in determining the output metric(s) of interest, e.g. for diagnosis, classification, prognosis, etc. purposes. For example, voxel-based morphometry techniques may be used to analyze e.g. MRI images of the brain, which is advantageously easy to use and/or simple. Spatial normalization techniques may be used to reduce the impact of normal variation in size and/or shape (and/or other large-scale variability) of the brain (which is, inter alia, also commonly applied in fMRI and/or PET analysis, and therefore may be considered to apply the processing of the functional imaging in embodiments of the present invention as well). This allows (normalized) white matter and/or grey matter quantification in the regions of interest, to determine local deviations from the reference (healthy) brain.

For example, migraine-with-aura patients may show less grey matter volume in the (left) inferior temporal gyrus, (right) frontal gyrus and/or (right) cerebellum, compared to migraine-without-aura, which may therefore be used for classification of these two types of migraine. Versus healthy subjects, migraine patients may show less grey matter volume in the (right and/or bilateral) cerebellum (and/or left cerebellum crus I), (left) post-central gyrus, (left) pre-central gyrus, (right) inferior and/or superior frontal gyrus, (left) superior/ medial and/or (right) inferior/middle frontal gyrus, (left) Brodmann areas 20-22, (left) fusiform gyrus, (right) parahippocampus, insula and/or the (left) lingual gyrus, and/or more grey matter volume in the (right) superior parietal gyrus and/or the (left) thalamus. Therefore, such differences in morphometrics may be used for the purposes of migraine detection (e.g. diagnosis). A substantial reduction of grey matter volume in the cerebellum, frontal and temporal gyrus may for example be indicative of the migraine condition (vs. healthy subjects and/or patients with other similar symptoms, e.g. cluster headache patients). Likewise, a reduced volume of the fusiform gyrus (and/or cingulate cortex) may be related to hyperactivity in pain responses. Volume of the cingulate cortex may also be linked to progression of the condition, e.g. from an episodic to more chronic disorder. Such reduced GM volumes in combination with abnormal activity and/or functional connectivity, e.g. increased activity (vs. a normal reference value), as detectable by functional imaging (e.g. in the fusiform and/or cingulate cortex) may be used as a strong indicator for the migraine condition. It is also noted that morphometry may be particularly useful for quantifying (in combination with other, functional, metrics or as such) progression of the disease, since variations in grey matter volume may originate from, e.g., repeated ischemia caused by the migraine (e.g. during attacks, but possibly also by changes in blood flow in the interictal phase); not necessarily precluding other mechanisms of migraine-related neurotoxicity.

Where reference is made to laterality, it will be understood that brain structures may be differently organized in some patients compared to others, e.g. depending on handedness (without limitation thereto), and explicit reference to e.g. a left-side effect does not preclude detection of a similar effect in the opposite side of the brain (or bilateral detection). For example, handedness may influence differences in lateralization of language areas as well as dominance of motor and somatosensory cortices, without necessarily being limited thereto. References to laterality may therefore be considered optional (but not necessarily so, depending on the specific implementation choices of an embodiment), and merely indicate the preferred and/or most likely anatomical side where the effect of interest may be observed. This equally applies to the discussion of regions of interest hereinbelow, e.g. relating to detectable activity patterns and/or functional connectivity patterns. For example, lateralization of a region or regions of interest may be determined in a patient-specific manner by functional connectivity analysis, dedicated functional tests (fMRI paradigms) and/or tractography analysis, e.g. such that the lateralized region(s) of interest may be (optionally) used in further analysis to determine the activity measure(s).

A machine learning method may offer many advantages, for an integrative combination of various sources of information (e.g. in determining the output values and/or activity measures) with subtle relevancy and possibly widely varying levels of noise and/or errors, e.g. when morphometric information is also taken into account. This is not limited to embodiments which (optionally) use morphologic information, since, as discussed hereinabove and further hereinbelow, functional information from many regions of interest in the brain may contribute some subtle and/or (e.g. region-per-region) limited information, such that the best results might be obtainable by using the power of machine learning to distill the output of interest (e.g. the migraine score or other related output) from the wide array of available information, which is potentially very noisy and/or close to the threshold of detection.

As a further remark, it is also pointed out that a machine learning algorithm may easily adapt to scenarios in which different sub-populations of patients show different activation patterns, without requiring an explicit definition of these sub-populations or even knowledge of the existence of such partitioning of the patient population, and may output, for example, a migraine probability score (e.g. for use in assisting diagnosis) that is substantially accurate regardless of which sub-population the patient corresponds to. In a more conventional approach, e.g. using a weighted combination of activation measures for a corresponding list of predetermined brain regions, in which the weights are e.g. handcrafted and tuned to the desired result, properly taking different properties of different sub-populations into account to improve the output metric(s) would require detailed knowledge of their distinguishing properties (and, obviously at the very least, knowledge of the existence of such sub-populations).

The at least one region of interest (for determining the activity measure/measures, which, for the sake of simplicity, may also refer to e.g. functional connectivity measures, and/or may also apply to morphometric analysis) in the brain may be linked to pain signal processing and sensation, attentional processing, executive function and/or sensory processing (e.g. visual, auditory, tactile, olfactory and/or other senses), resting wakeful state maintenance, memory function, emotional processing, sensorimotoric function and/or other higher-level brain functions.

Brain networks which may be particularly of interest, e.g. as triggered by provided stimuli and/or in natural coordinated activity between brain regions (e.g. in resting state), may include the salience network (midcingulo-insular network), the central executive network (frontoparietal network, or lateral fronto-parietal network) and/or the default mode network. Thus, dysfunctions in attentional processing (e.g. the selection of attended vs. unattended events and the ensuing shifts and/or continued tracking of the brain's focus), the brain's resting state (accommodating wakeful rest, mind-wandering, daydreaming, etc.) and executive function (working memory, problem-solving, ...), each in isolation and/or in their interaction, may be particularly of interest. Furthermore, alternatively or in addition to the aforementioned higher-level networks, the sensorimotor network, e.g. which is involved in coordinating motor tasks, may be of interest, and/or networks activated by painful and/or sensory stimuli (e.g. visual stimulation).

The activity measure may thus comprise measures of activity in regions of the salience network and/or correlations (in the broad sense, i.e. not necessarily limited to linear correlation) between activity in these regions. The relevant brain area(s) may include: the (e.g. anterior) insula, the (e.g. dorsal) anterior cingulate cortex, the inferior parietal cortex, the (e.g. right) temperoparietal junction, the lateral prefrontal cortex, the amygdala (and/or sublenticular extended amygdala), the putamen, the ventral striatum, the (e.g. dorsomedial) thalamus, the hypothalamus and/or the substantia nigra and/or ventral tegmental area. The relevant brain areas may include the frontal eye fields, the superior parietal lobe and/or the intraparietal sulcus, forming the dorsal part of the network, the temporal parietal junction (the intraparietal junction and/or superior temporal gyrus and/or sulcus), and the ventral frontal cortex (e.g. the middle and inferior frontal cortex). The predetermined brain region(s) may thus comprise any one or combination of the aforementioned relevant brain areas and/or any one or combination of subregions thereof. For example, activity in these regions (and/or correlations of activity within or between them) may be tested in response to an attentional processing task, such as a Posner or Posner-related cueing task.

For example, stimuli in accordance with a voluntary and/or reflective visual spatial orienting task, e.g. in which the patient is (mentally) orienting voluntarily to (visually) central cues and/or reflexively to (visually) peripheral flash cues (e.g. displayed markers, arrows and/or other symbols), may be provided to test brain activity in regions related to the salience network and regions/networks functionally connected thereto, and/or to test functional connectivity within and/or between such brain regions. The ventral frontopariental (attentional) network and/or the (typically right) temporal parietal junction may generally show reduced activity in migraineurs vs. healthy (or non-migraine) control subjects, e.g. which may be considered to be generally indicative of reduced suppressive capability of unattended events (and, thus, may also be associated with hypersensitivity symptoms as often encountered in migraineurs).

Resting state analysis of the salience (and/or other) network may also be used, e.g. to detect changes (e.g. of migraine patients vs. healthy subjects) in functional connectivity (e.g. using regional homogeneity as an indicative measure) in the putamen, the orbitofrontal cortex and/or the thalamus. Such changes may also be observed in the brainstem and/or secondary somatosensory cortex (e.g. lining to lower-level sensory and/or nociceptive processes).

The activity measure may comprise measures of activity in regions of the central executive network and/or correlations (in the broad sense) between activity in these regions. The relevant brain area(s) may include: the dorsolateral prefrontal cortex, the posterior parietal cortex (e.g. near/around the interparietal sulcus), the rostral lateral prefrontal cortex, the anterior inferior parietal lobule, the middle cingulate gyrus, the dorsal precuneus, the posterior inferior temporal lobe, the (e.g. dorsomedial) thalamus, and/or the (e.g. head of the) caudate nucleus. The predetermined brain region(s) may thus comprise any one or combination of the aforementioned relevant brain areas and/or any one or combination of subregions thereof. For example, activity in these regions (and/or correlations of activity within or between them) may be tested in response to a cognitive processing task.

The activity measure may comprise measures of activity in regions of the default mode network and/or correlations (in the broad sense) between activity in these regions. The relevant brain area(s) may include: the medial prefrontal cortex, the posterior cingulate cortex (and/or the precuneus), the angular gyrus, the dorsal medial prefrontal cortex, the temperoparietal junction, the lateral temporal cortex, the anterior temporal pole, the hippocampus, the parahippocampus, the retrosplenial cortex and/or the posterior inferior parietal lobe. The predetermined brain region(s) may thus comprise any one or combination of the aforementioned relevant brain areas and/or any one or combination of subregions thereof. For example, activity in these regions, and (particularly) correlations of activity within or between them, may be analyzed using resting state functional magnetic resonance imaging techniques.

The activity measure may comprise measures of activity in regions of the sensorimotor network (pericentral network) and/or correlations (in the broad sense) between activity in these regions. The relevant brain area(s) may include the lateral parietal lobe, e.g. the postcentral gyrus (e.g. the somatosensory region/regions of the primary somatosensory cortex), the posterior frontal lobe, e.g. the precentral gyrus (e.g. region/regions of the primary motor cortex), the supplementary motor area and/or the superior temporal gyrus (e.g. the auditory cortex). The predetermined brain region(s) may thus comprise any one or combination of the aforementioned relevant brain areas and/or any one or combination of subregions thereof. For example, activity in these regions may be analyzed in relation to other networks, e.g. concomitantly triggered by a suitable task targeting one or more different brain systems, and/or directly stimulated by (per instruction/cue to the patient) finger-tapping or similar motor tasks and/or by tactile stimulation (e.g. in combination with nociceptive stimulation, e.g. simultaneously or by variations in the stimulation intensity).

For example, a functional connectivity analysis of brain networks, such as the default mode, central executive and/or salience network (e.g. forming the triple-network model), and/or the sensorimotor network, may be used to determine the activity measure(s) (and/or an element/subset thereof) for diagnostic (e.g. a migraine index or score indicative of the likelihood of the patient being a migraineur) and/or classification (e.g. differential diagnostic) purposes, e.g. a metric indicative of the patient being a migraine-with-aura vs. migraine-without-aura patient. A measure (or measures) of functional connectivity within (and/or between) the (e.g. left) angular gyrus, (e.g. right) precentral gyrus, (e.g. right) postcentral gyrus, (e.g. left) supramarginal gyrus and/or (e.g. right) insular cortex may generally correlate with (in the broad sense, e.g. higher values being indicative of a higher "score" or likelihood of) the patient being a with-aura type migraine patient as opposed to a without-aura type migraineur. Thus, connectivity between regions associated with pain processing, salience, sensorimotoric function and executive function may be out of balance and/or reorganized with respect to the average healthy person, such that objective measurements of such connectivity (and/or activation patterns) can be advantageously used in accordance with embodiments of the present invention. Tests of connectivity (and/or activity) in the aforementioned regions may, for example, be complemented with (without limitation thereto) activity (and/or connectivity) measured in the visual cortex (e.g. in combination with suitable visual stimuli to induce such activation), which may also contribute some discriminating power for the purposes of classification between these main types of migraine, as also discussed further hereinbelow.

For example, functional connectivity within the (e.g. bilateral) central executive network and/or salience network may substantially deviate from healthy subjects (e.g. migraineurs may show aberrant functional connectivity in the CEN and/or SN). Considering the inter-network connectivity (as may be expressed by the activity measure or component thereof), a greater functional connectivity between the insula and the default-mode network (DMN) and/or (e.g. right) central executive network (CEN) may be indicative of migraine (e.g. as opposed to different conditions, e.g. healthy subjects and/or patients experiencing different types of headaches). Likewise, a greater connectivity between the DMN and the CEN (e.g. right) may be indicative of migraine, and, furthermore, this increased connectivity may (e.g. positively) correlate (but not necessarily linearly) with the duration of migraine, such that this association may be used for (as a contributing element or as basis for a measure as such) staging the severity and/or level of migraine, e.g. to quantify the level of disruption in the brain networks associated with long-term migraine (e.g. potentially caused by repeatedly experiencing migraine attacks over time).

The activity measure may comprise measures of activity in regions associated with vision, for example brain activation in response to visual stimuli, and/or correlations (in the broad sense) of such activation with other regions (or within such region). Regions of interest may include the primary visual cortex, the lateral geniculate nucleus and/or the visual motion-responsive middle temporal cortex (e.g. human brain area hMT+). For example, (e.g. positive) activation of these three regions in response to visual stimuli may be associated with migraine, e.g. a greater activation compared to healthy subjects.

It is noted that specific (e.g. threshold and/or normalization and/or scaling) values (e.g. for the activity measure/measures) may depend on the specific type of imaging scanner, imaging protocol and stimulation paradigm, and are therefore difficult to determine for a general use case. However, robust results can easily be obtained by calibrating the methodology with results obtained from a set of healthy subjects (and/or a set of known migraineurs, optionally with different, known, types of migraine). Furthermore, differences in the observable activation in these regions (in combination or in a subset thereof) may be used to differentiate migraine-with-aura patients from migraine-without-aura. For example, hyperresponsivity to visual stimuli in migraine patients may be associated with a greater activation in the (e.g. primary) visual cortex (and/or middle temporal gyrus and/or lateral geniculate nucleus), and thus may be used to differentiate patients.

Furthermore, brain regions (and/or their connections, i.e. the functional network or networks formed by such regions) involved in nociception and the processing of pain sensations (and further upstream processes) may be particularly of interest, e.g. as may be induced by intense chemical, mechanical and/or thermal stimulation (and, thus, may be conveniently triggered by corresponding stimuli during the functional imaging procedure). The lower-level and/or higher-level processing and perception of such signals, e.g. the brain regions affected by, controlling, contributing to and/or modulating physiological and/or behavioral responses to pain, may show different activation patterns in migraine patients relative to healthy subjects, and/or may differ between different types of migraine. For example, the insula (also insular cortex, insular lobe), or subregion(s) thereof (incl. left/right and/or bilateral, anterior and/or posterior), may be a region of interest, in view of its connection to awareness of pain and the judging of pain intensity, and its involvement in consciousness and/or perception. For example, the insula may show activation in response to nociceptive stimulation, which may be used for diagnostic and/or prognostic purposes (e.g. using PET and/or fMRI, without limitation thereto).

The activity measure may comprise measures of activity in (a) region(s) of interest associated with pain signaling (nociception) and various functions associated therewith, e.g. transmission of pain signals, hormonal responses to pain, intensity of the pain experience, painful memory encoding and recollection, and/or the perception of pain. Regions that may provide a functional link to (any) other(s) of the aforementioned networks may also be particularly relevant, such as connections to the salience network, default mode network and/or central executive network. The relevant brain area(s) may include the periaqueductal gray, the thalamus (e.g. thalamic intralaminal nuclei), the amygdala, the hippocampus, the hypothalamus, the cingulate cortex, the prefrontal cortex and/or the insula. The predetermined brain region(s) may thus comprise any one or combination of the aforementioned relevant brain areas and/or any one or combination of subregions thereof. For example, activity in these regions (and/or correlations of activity within or between them) may be tested in response to noxious stimulation, e.g. by ammonia, capsaicin, heat, cold and/or other chemical, mechanical and/or thermal means of delivering a pain trigger signal to the brain.

For example, migraine patients exposed to (e.g. thermal) pain stimulation may show different activation patterns (e.g. interictally) compared to healthy subjects, for example greater activation in pain-facilitating regions and lower activation (or relative deactivation) in pain-inhibiting regions. This migraine 'signature' pattern may be detected in various brain regions as discussed hereinabove, e.g. involved in pain signaling, sensorimotoric function and/or salience (without necessarily limiting thereto). For example, the activation measure(s) may be determined for the following regions and/or a subset thereof (and/or a combination of subregions thereof): the temporal pole, the parahippocampal gyrus, the (middle and/or anterior) cingulate cortex, the lentiform nuclei, the fusiform gyrus, the subthalamic nucleus, the hippocampus, the (primary and/or secondary) somatosensory cortex, the dorsolateral prefrontal cortex, the precentral gyrus, the superior temporal gyrus and/or the brainstem. For example, a greater activation (e.g. fMRI and/or PET) may be detected for migraineurs relative to healthy subjects in the amygdale, the insula, the temporal pole, the superior temporal gyrus, the cerebellum and/or the rostral pons. For example, in response to painful heat stimulation, increased activity (e.g. vs. healthy subjects) may be detected in the temporal pole and/or the parahippocampal gyrus.

In the brainstem, the medulla oblongata may be particularly targeted as a region of interest (for determining the activity measure), and/or, more specifically, the spinal trigeminal nucleus (or subregion or superset thereof). Stimulation of the nociceptive system, e.g. using ammonia gas (without limitation thereto, e.g. other noxious chemicals, other types of pain stimuli and/or direct trigeminal stimulation may also be considered), and determining the activity measure (or as an element of the activity measure) for a region of interest comprising the spinal trigeminal nucleus may be used to determine a predictor for a next migraine attack, e.g. in view of a correlation between brain activation (e.g. strength and/or extent of activation) in said region with time remaining until the next migraine episode.

The cingulate cortex (and/or parts thereof) may also be of interest, in view of its connection to pain and memory, as well as its possible shared connection to the central executive network and/or salience network. A further region of interest, e.g. in view of a possible connection to the central executive network and/or salience network, and to pain perception in general, may include the thalamus (and/or sub-regions thereof). Likewise, the amygdalae (and/or subregions thereof) may be of interest in view of association with pain, emotion, encoding of memory and/or a link to the salience network. For example, the amygdala may show (PET, without limitation thereto) activation during nociceptive stimulation. The hippocampus (and/or sub-regions thereof) may also be of interest, e.g. providing a possible link to the default mode network. Lower-level processes involved in pain perception and/or hormone-mediated pain modulation (and/or serotonin pain modulation) may involve the periaqueductal gray, the thalamus (and/or parts thereof), and/or the hypothalamus, which therefor may also be (a) region(s) of interest. Also, the pons (and/or parts thereof, e.g. one or more rostral pons regions) may be of interest, in view of its relay function and/or involvement in the sensation of pain (e.g. facial pain). For example, (a region of) the rostral pons may show activation consistent with being a potential migraine source or potentiator (e.g. when triggered by external nociceptive stimuli causing the onset of a migraine attack).

During a migraine attack (even though embodiments may preferably use imaging in the interictal phase for practical reasons; not necessarily excluding embodiments using ictal phase imaging), increased activity in the posterior thalamus may be detected, e.g. and may be used in accordance with embodiments for diagnosing migraine vs. other conditions. For example, this region may play a role in creating extracephalic allodynia, a sign of central sensitization during migraine attacks (and may potentially contribute to a differentiation between different types of migraine and/or different specific pain symptoms in migraine patients).

For example, in the premonitory phase (leading up to a headache attack), activity in the posterior hypothalamus may be increased (e.g. possibly explaining fatigue and/or irritability in this pre-headache phase; e.g. as may be detected by PET and/or fMRI in this attack prelude phase), as well as in other related areas (e.g. involved in pain, sensory and/or motoric function), such as the periaqueductal gray, the midbrain tegmentum, the dorsal pons, the posterior thalamus (e.g. see also hereinabove) and/or various cortical areas (see e.g. the discussion hereinabove with respect to the triple network model regions).

In view of typical symptoms of migraine comprising intense headaches, a region of interest (which may be relevant in various stages of the migraineur, e.g. activity may be detected in the interictal phase, but also ictal) may comprise the spinal trigeminal nuclei (and/or combinations, subsets and/or subsets thereof, e.g. the subnucleus caudalis associated with the transmission of nociception from the head). Since the nucleus projects to the ventral posteriomedial nucleus in the (contralateral) thalamus, it is noted, again, that also the thalamus and/or subregions thereof may have a central role in the processes of interest. For example, the activation (e.g. observed by fMRI, e.g. during nociceptive stimulation) has been observed to correlate with the time until a next migraine attack, such that this activation may be tested and used for prediction (and/or staging, differential diagnosis, migraine diagnosis and/or other related measures).

Therefore, activity in regions such as the spinal trigeminal nuclei (e.g. when stimulated by nociceptive stimuli), the medulla oblongata and/or the brainstem in general (e.g. the periaqueductal gray, the dorsal pons, the substantia nigra, the red nucleus and/or the ventral tegmental area, without limitation thereto) and/or other regions (see e.g. the discussion hereinabove relating to indicative activity in the premonitory phase, without limitation thereto) may be used to predict the onset of a next migraine attack and/or to estimate the expected frequency of attacks (and, potentially related thereto, the severity of the migraine case and/or the duration of the pre-existing migraine condition). For example, the brainstem (and/or regions therein) may play an important role in mediating the onset of a migraine attack. The hypothalamus may also be involved in triggering migraine, and thus also used for calculating such predictive (and/or other) metrics. Furthermore, the extent of rs-fcMRI abnormalities (e.g. in/between regions of the triple-network model; e.g. vs. healthy subjects) may also correlate with headache frequency and/or the number of years of the pre-existing migraine condition, and thus may be used in accordance with embodiments.

The method comprises outputting 15 at least one value, based on said activity measures, that is indicative of, e.g. representative of, e.g. that expresses, a (patient-specific) migraine score, e.g. for diagnosis (e.g. with respect to healthy subjects and/or other disorders), assessment, and/or follow-up purposes and/or to quantify the severity of the migraine case, a classification score (e.g. to express whether the patient is a migraine-with-aura or migraine-without-aura patient, without limitation thereto; not necessarily as a binary or discrete value, e.g. a classification may also be expressed in terms of probability or odds), and/or at least one prognostic value relating to the migraine condition (e.g. a prediction of future evolution of the condtion, a prediction of expected efficacy of one or more treatments, ...). For example, the latter may comprise a ranking (e.g; in terms of projected suitability and/or efficacy) of candidate treatments, a selection of candidate treatments, and/or one or more proposed treatment plans.

Thus, activity in brain regions such as discussed hereinabove and/or functional connectivity between (and/or within) such regions (and/or morphometric measures relating to such regions) may be used to diagnose a migraine, e.g. to distinguish the migraineur from patients experiencing headaches and/or other symptoms due to different diseases and/or conditions.

For example, an imbalance between pain-inhibiting and pain-facilitating brain processes may be detected as a telltale indication of the migraine condition, e.g. a sensitization of the brain to further migraine attacks.

As noted hereinabove, specific activation patterns (and/or functional connectivity patterns) may also (additionally or alternatively) be used to differentiate migraine conditions, e.g. to classify migraines based on their specific type, e.g. migraine-with-aura and migraine-without-aura. For example, visual stimuli (and/or other sensory stimuli) may be used to detect patterns of a hyperexcitable occipital cortex (e.g. indicating a predisposition to cortical spreading depression) and/or other regions, e.g. involved in visual, sensory and/or attentional processing (e.g. the salience network). Hypersensitivity (for classification of the migraine type and/or gauging the severity and/or stage of the case) may also be associated with enhanced activation in the temporal pole during an attack (or shortly before), which may also be used advantageously for gaining insight in the patient's condition.

It will also be understood that objective diagnosis, classification and/or prediction metrics may be used to guide therapy and/or management of the condition, e.g. to determine an expected efficacy of a specific therapy (e.g. drug or drug combination) for a specific patient based on the objective metrics determined by functional imaging. Furthermore, repeated use of a method in accordance with embodiments to establish such metric(s) may allow the condition of the patient to be followed up in an objective manner, e.g. to detect when the condition alleviates or worsens and/or to prognose a further evolution. The latter may also allow treatments to be planned proactively, e.g. to anticipate on a potentially negative outlook when no corrective measures are taken.

The method may be adapted to monitor and/or determine the effect of stimuli on a patient, to evaluate the evoked physiological effect of such stimuli by functional imaging (and/or to quantify inherent processes, e.g. in resting state), to quantify these (provoked and/or inherent) effects, and/or to determine one or more measures, e.g. score(s) and/or rating(s), expressing (a) characteristic(s) of the migraine condition, such as a probability (or related measure, e.g. likelihood) that the patient suffers from migraine, a classification into different types of migraine condition (e.g. a score for each possible class), a measure indicative of the severity of the migraine, a predictive measure of frequency of migraine attacks and/or of time until a next attack. This may also include a score or rating (or similar indicative measure) for different treatment modalities to predict their likely efficacy and/or suitability, e.g. of different drugs, lifestyle interventions, therapeutical approaches, and/or combination treatments. Thus, a personalized therapy plan may also be generated based on such outputs, e.g. as a computer-aided suggestion to the attending physician. The output information may be used for clinical support, e.g. to enable a better classification of migraine at the level of patient, a grading of its severity and/or, consequently, to improve migraine management by tailoring migraine therapy to the patient's needs and response.

As already mentioned, additional information may be obtained and taken into account, e.g. in determining the activity measure(s) and/or in determining and outputting 15 the at least one output value. This may include additional information for assessment of the effect of the stimuli, e.g. face reaction data, eye movements, pupil dilation, muscle reaction and/or physiological reactions, such as breathing rate and/or heart rate. This may also (additionally or alternatively) comprise a score(s) based on a clinical diagnosis scale (e.g. an a-priori evaluation of the patient by an attending physician), self-reporting information (e.g. from a patient's migraine diary), and/or results of psychometric tests (e.g. cognitive tests). Thus, longitudinal data of a patient may be used in conjunction with (functional) imaging data of the patient to create an individual migraine index and/or other metrics related to the patient's migraine condition. Such individual migraine index (and/or other output value/values) may provide an objective, quantitative tool to measure, and follow-up on, the effect of migraine on the specific patient.

It will be understood that some of these examples of further supporting data can be obtained in real-time and/or at a sufficient frequency during the functional image (e.g. fMRI timeseries) acquisition, e.g. may be directly taken into account in the fMRI (and/or PET, ...) analysis, while other examples may only allow for infrequent or even a single time sample, e.g. a grading of a psychometric test (e.g. of executive function, attentional processing, ...). Nonetheless, such information that is poorly resolved in time (e.g. to directly guide the analysis of the functional imaging data) may still be taken into account in the end result, i.e. the output value(s), for example in a weighted combination (of activity measures and such other data), as parameters of a predetermined function for combining the data and/or as inputs to a trained machine learning model.

For example, a multimodal assessment using neuroimaging (fMRI, MEG, EEG and/or PET, ...) together with standardized clinical scales based on, e.g., self-reporting information may be used to provide a more robust evaluation of the migraine condition.

Sessions in which the method is applied to evaluate a patient may be repeated over time, and previous results (e.g. the activity measures and/or previous output values) and/or an initial value (e.g. from a first session) may be taken into account to determine a progress of the patient and/or to prognose a future progress. This may be done by a deterministic algorithm, e.g. using time series analysis, regression techniques or the like and/or by a machine learning technique, e.g. using the data from different sessions of the method for the same patient, e.g. based on fMRI data from different sessions, as input to obtain a further output, e.g. a pattern index, trained on retrospectively observed improvements and/or changes in the patient's condition in a training set of patients. It will be understood that an fMRI (or PET, ...) analysis algorithm may also be adapted to take previous results into account if available, e.g. a machine learning algorithm may be trained to receive further inputs representative of one or more previous sessions to take the patient's history into account.

The at least one output value that is determined and outputted 15 may comprise a personal migraine index, e.g. a score indicating the likelihood and/or estimated severity of the migraine case. For example, objective data (i.e. the activity measure/measures) may be extracted from analysis of MRI brain scan data (but also, additionally or alternatively, PET and/or other functional imaging modalities), in which this information may determine and/or quantify the effect of migraine on various brain area(s) as discussed hereinabove. The functional (MRI) data may include resting state fMRI and/or fMRI analysis of activity (and/or connectivity) in response to stimuli (e.g. using a block and/or event-related paradigm), such as cognitive tests, sensory stimuli, nociceptive stimuli, ... (cf. as discussed hereinabove). Rs-fcMRI and conventional stimuli-response fMRI may also be combined in embodiments of the present invention. The task paradigm (for a conventional stimuli-response fMRI protocol) may also be used to obtain side-information to take into account, such as cognitive responses of patient, measurements of attentional shifts (e.g. eye tracking) and/or other side-information gathered during the stimulation and imaging protocol.

In addition to taking the determined activity measure(s) into account, objective and/or subjective side-information may be taken into account as well, e.g. by a weighted combination (but not necessarily merely a weighted summation) or, possibly preferably, a trained machine learning algorithm. Subjective data may relate to the patient's experience before (triggers), during (pain) and/or after a migraine attack, but is not necessarily limited thereto. The subjective data may, for example, include information related to the experience of pain, e.g; provided by patient on how he/she experiences migraine pain. For example, a standard questionnaire may be used to obtain such data, and/or a variation thereof, e.g. in which the experience of pain is quantified and/or ranked by the patient (e.g. on a scale from 0 to 10, where 0 represents no pain at all and 10 is representative of the most terrible pain as subjectively experienced or imaginable by the patient). The migraine index may thus take such information into account in its input (as subjective side-information) and/or in generating a (e.g. component of the) output, e.g. a personal severity migraine Index. However, by taking objective information into account as well, this output value may be more objective than a mere result of self-reporting, e.g. may allow for normalization and/or scaling in view of detectable, e.g. pain-related, brain (functional and/or morphologic) abnormalities. The output metric(s), e.g. a personal migraine index and/or personal severity migraine index, may be used to rank the individual migraine pain, the personal experience of migraine pain and/or to gauge the level of individual pain acceptance.

The personal migraine index (in general, the output values of the applied method) may be used for clinical support, e.g. to provide a reliable diagnosis and/or individual classification of migraine. The Information may also be used for optimization and personalization of treatment. By follow-up examinations (e.g. repeated application of the method) longitudinal (objective and/or subjective) data may become available, and thus used to further improve the migraine index and/or to express the evolution of the index (and thus, the patient's condition). Thus, the migraine index may be used to evaluate the course of migraine (worsening or improving, i.e. the progression of the migraine case), to calculate (and/or predict) changes over time and/or to monitor the effect (and/or effectiveness) of a therapy and, if needed, to further adjust the treatment plan to the individual patient, e.g. to optimize and/or tailor a current treatment and/or to determine the (estimated) most optimal treatment, which advantageously may avoid patients going through different (ineffective and/or sub-optimal) treatments before establishing a suitable, effective and/or most optimal therapy approach. Likewise, the information obtained by applying the method in accordance with embodiments may be used to not only evaluate the response to a treatment and to develop a further treatment strategy, but also to evaluate the risk of migraine chronification.

The information collected over time, e.g. by repeated application of a method in accordance with embodiments, may be analyzed by conventional means (e.g. regression methods) and/or machine learning, e.g. to provide a pattern index. Such long-term analysis, e.g. the pattern index, may be used as an indication of the patient's improvement (or decline, or generally evolution and/or changes in the condtion). For example, the analysis results may express neurological changes (neurorehabilitation) as compared to an initial (reference) state. Such information may also be useful to study interindividual variations in migraine disease characteristics and/or to determine why and when migraine occurs.

Even though, as described hereinabove, the activity measure(s) (and/or functional connectivity, morphometric data, ...) relating to specific regions of interest in the brain may be used as such to determine, e.g. as the output value or component thereof, a measure of the frequency of migraine attacks and/or predictions of the timing of future attacks (e.g. a predicted time to the next attack), longitudinal data (e.g. repeated applications of the method and analysis of evolution of the activity measure/measures and/or output value/values, and/or longitudinal side-information, such as patient migraine diary information, records of previous attacks, changes over time in objective measurements and/or subjective descriptors relating to stress and/or lifestyle (e.g. resting heart rate, heart rate variability, breathing rate, diet changes, work-related stress assessment, cortisol measurements, ...) may be used as well (or in combination) to determine such migraine attack frequency and/or timing (retrospective and/or predictive). For example, pattern analysis of the available data (which may include the objective imaging-based data, values derived therefrom and/or such longitudinal side-information) may be used to accurately detect an increased (or increasing) sensitization to migraine attacks, the early prelude of a migraine headache attack and/or a sequence of different phases (and current/next phase) through which the migraine evolves (e.g. cycles).

The at least one output value provided 15 by the method may also comprise an analysis of the effect of different stimuli and/or stressors, e.g. potential migraine triggers, for indicating which type of and/or characteristics of sensory stimuli may be a risk factor in triggering a migraine attack. Additionally or alternatively to a more conventional analysis of the effect of different types (and/or parameters) of (e.g. sensory) stimulation during the functional imaging, machine learning techniques may be used to identify personal migraine triggers for the patient, e.g. taking the activity measures in the brain in response to different stimuli into account, but optionally also further side-information, such as reported lifestyle events of the patient, self-reporting information (migraine diary, food logs, stress ratings, ...), etc. Thus, the output value(s) may be used for individual guidance of the patient, e.g. to prevent or reduce the occurrence of migraine attacks by advising lifestyle changes, food selection, stress reduction and/or avoidance of specific triggers and/or stressors.

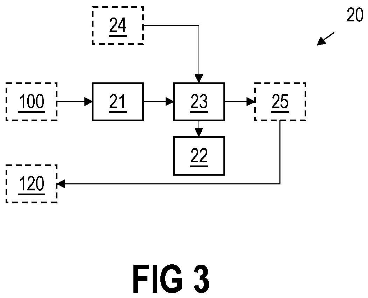

Referring to Fig. 3, in a second aspect, the present invention relates to a device 20, e.g. a computer system (e.g. the computer system 122 of an MRI system 100) and/or specifically adapted and/or programmed processing hardware, e.g. a device for executing a method in accordance with embodiments of the first aspect of the present invention. The device is adapted to analyze neural activity in the brain of a patient by functional brain imaging to diagnose, assess, follow-up and/or classify a migraine condition of the patient. The device comprises an input 21 for receiving functional brain imaging data, e.g. one or more images of the patient's brain, e.g. tomographic images, e.g. volumetric 3D images. The device comprises an output 22 for outputting at least one value, and a processor 23 for determining at least one activity measure indicative of brain activity in and/or between at least one predetermined region of interest in the patient's brain based on said functional brain imaging data. The processor is adapted to determine and output said at least one value, via said output 22, based on the at least one activity measure, This at least value is representative of a patient-specific migraine score indicating a probability and/or severity of the migraine, a migraine type classification, a prognostic value to predict frequency of migraine attacks and/or an estimated time until a next attack and/or a future evolution of the migraine condition, and/or an assessment of the influence of different types of migraine triggering events and/or of the predicted efficacy and/or suitability of different treatments.

The processor may be adapted to determine the activity measure representative of brain activity in, and/or expresses a relationship of local activity within and/or between one or more of, said predetermined region(s) of interest. The at least one predetermined region of interest may comprise a brain region or brain regions involved in pain signal processing and/or pain sensation, attentional processing, executive function, sensory processing and/or sensorimotoric function, resting wakeful state maintenance, memory function and/or emotion.

A device in accordance with embodiments may comprise a further input 24 for receiving further imaging, physiological, medical, psychometric, behavioral, lifestyle, self-reported pain experience and/or diagnostic data pertaining to said patient. The processor may be adapted to take the further data into account in determining the activity measure and/or in determining the at least one value.

The device may comprise a further output 25 for controlling at least one stimulus source 120 (which may be external and/or included in the device, and/or the device may combine interfaces to one or more external stimulus sources and one or more included stimulus sources). The processor may be adapted for providing a plurality of stimuli to the patient at different points in time via the further output 25 and the at least one stimulus source 120 during the acquisition of the functional brain imaging data so as to induce the brain activity of interest (directly and/or indirectly).

The plurality of stimuli may be provided in accordance with a blocked-trial functional imaging paradigm and/or an event-related functional imaging paradigm (or a hybrid combination thereof). The at least one activity measure (or component thereof) may be determined by processing the functional brain imaging data in accordance with this (stimulation/imaging) paradigm using a model for taking the timing of the stimuli and of the image acquisitions into account. The model may comprise a general or generalized linear model, a statistical parametric mapping model (e.g. a SPM or similar analysis method may be used) and/or a trained machine learning model. The at least one value may also be determined by a (or the) trained machine learning model, e.g. based on the at least one activity measure (which may be determined by another trained machine learned model or by a more conventional, e.g. statistical analysis, method), or directly from the functional brain imaging data (or intermediate features derived therefrom). In other words, the activity measure may also be implicitly encoded in an intermediate stage of a machine learning model for determining the output value(s).

The stimulus source(s) may comprise a nociceptive stimulator, a sound source, a light source and/or display (for example, a monitor, a projector and projection screen, a HUD device, ...), a haptic and/or tactile stimulator, an olfactory stimulator (e.g. for time-controlled release of pugnant and/or odorous chemicals), a thermal stimulator (e.g. a heater and/or cooler) and/or a(nother kind of) sensory stimulator.

The device may be adapted to provide nociceptive stimuli, and to determine brain activity in (and/or correlated activity within/between) one or more regions of the pericentral sensorimotor network and/or one or more brain regions affected by, controlling, contributing to and/or modulating physiological and/or behavioral responses to pain, so as to detect an imbalance in brain activation between pain-facilitation and pain-inhibition.

The device may be adapted to provide stimuli in accordance with a Posner cueing task and/or another attentional processing task, and to determine activity in/between one or more regions of the midcingulo-insular salience network to detect facilitated hypersensitivity by dysfunctional suppression of non-attended events and/or other dysfunctional attentional processing in the selection of, mental shifting between and/or continued focus maintenance on attended versus unattended events provided in said task.

The device may be adapted to provide stimuli in accordance with a cognitive processing task, and to determine activity in/between (a) region(s) of the frontoparietal and/or lateral fronto-parietal central executive network.

Different sets of sensory stimuli may be provided by the device at different times and/or in different combinations (with different sensory modalities, stimulation conditions, stimuli content and/or stimulation characteristics, ...). The at least one activity measure and/or the output value may be determined so as to score and/or rank the different types of sensory stimuli and/or thereto related stressors for their estimated potential to evoke, facilitate and/or trigger a migraine attack.

The stimuli may comprise visual stimuli, and activity in/between one or more regions of the visual cortex, the thalamus and/or other brain areas involved in vision may be determined, so as to detect abnormal activity in, and/or interaction within and/or between said one or more regions. The response to such visual stimuli may be used (by the processor) to determine (as output value or part thereof) a classification (e.g. discrete or as a probabilistic score) of the patient as a migraine-with-aura or migraine-without-aura patient.

Furthermore, the device may also be adapted to acquire functional brain imaging data in a resting state of the patient, without concomitant external stimulation, (or receive such data acquired in said resting state from an external source, e.g. an MRI scanner). The activity measure(s), or component thereof, may be determined by performing a resting-state functional connectivity imaging analysis so as to determine functional connectivity within and/or between region(s) of interest comprising one or more regions of the default mode network, the midcingulo-insular salience network and/or the fronto-parietal central executive network.

The processor may also be adapted to determine at least one morphometric value of one or more of said at least one predetermined region of interest based on said functional brain imaging data and/or on further imaging data of the patient's brain, and to take said at least one morphometric value into account in determining the at least one activity measure and/or the at least one output value.

Other features, or details of the features described hereinabove, of a device in accordance with embodiments of the present invention shall be clear in view of the description provided hereinabove relating to a method in accordance with embodiments of the present invention (and/or vice versa).

In a third aspect, the present invention relates to a magnetic resonance imaging system 120, cf. Fig. 1 and the discussion hereinabove, adapted to perform a method in accordance with embodiments of the first aspect of the present invention and/or comprising a device in accordance with embodiments of the second aspect of the present invention.

In a fourth aspect, the present invention relates to a computer-program product adapted to perform a method in accordance with embodiments of the first aspect of the present invention when executed by a computer, e.g. for implementing, by execution thereof, a device in accordance with embodiments of the second aspect of the present invention.

In a further aspect, the present invention relates to a diagnostic imaging workstation, e.g. an MRI workstation, , e.g. configured to view and/or process medical images, comprising a device in accordance with embodiments of the second aspect of the present invention.

## Claims

1. A device (20; 122) for analyzing neural activity in the brain of a patient by functional brain imaging to diagnose, assess, follow-up and/or classify a migraine condition of the patient, the device comprising:
an input (21) for receiving functional brain imaging data of the brain of the patient;
an output (22) for outputting at least one value; and
a processor (23) for determining at least one activity measure indicative of brain activity in at least one predetermined region of interest and/or between predetermined regions of interest in the patient's brain based on said functional brain imaging data, and for determining and outputting said at least one value, via said output (22), based on said at least one activity measure,
wherein said at least one value is representative of a patient-specific migraine score indicating a probability and/or severity of the migraine, a migraine type classification, a prognostic value to predict frequency of migraine attacks and/or an estimated time until a next attack and/or a future evolution of the migraine condition, and/or an assessment of the influence of different types of migraine triggering events and/or of the predicted efficacy and/or suitability of different treatments.

2. The device of claim 1, wherein said processor (23) is adapted to determine said at least one activity measure representative of brain activity in, and/or expresses a relationship of local activity within and/or between one or more of, said at least one predetermined region of interest, wherein the at least one predetermined region of interest comprises at least one brain region involved in pain signal processing and/or pain sensation, sensory signal processing and/or sensorimotoric function, attentional processing, executive function, resting wakeful state maintenance, memory function and/or emotion.

3. The device of any of the previous claims comprising a further input (24) for receiving further imaging, physiological, medical, psychometric, behavioral, lifestyle, self-reported pain experience and/or diagnostic data pertaining to said patient, wherein said processor (23) is adapted to take said further data into account in determining the activity measure and/or in determining the at least one value.

4. The device of any of the previous claims, comprising a further output (25) for controlling at least one stimulus source (120), wherein said processor (23) is adapted to provide a plurality of stimuli to the patient at different points in time, via the further output and the at least one stimulus source, during acquisition of said functional brain imaging data so as to induce said brain activity.

5. The device of claim 4, comprising said at least one stimulus source (120), the at least one stimulus source being adapted for providing the plurality of stimuli to the patient and the at least one stimulus source comprising a nociceptive stimulator for generating nociceptive stimuli, a sound source for generating auditory stimuli, a light source and/or display for generating visual stimuli, a haptic and/or tactile stimulator for generating haptic stimuli, an olfactory stimulator for generating olfactory stimuli, a heater and/or cooler for generating thermal stimuli and/or a sensory stimulator to generate other sensory stimuli.

6. The device of any of the claims 4 to 5, wherein said processor (23) is adapted to provide the plurality of stimuli, comprising nociceptive stimuli, and to determine the at least one activity measure indicative of the brain activity in and/or between the at least one predetermined region of interest comprising one or more regions of the pericentral sensorimotor network and/or one or more brain regions affected by, controlling, contributing to and/or modulating neural, physiological and/or behavioral responses to pain, so as to detect an imbalance in brain activation between pain-facilitation and pain-inhibition.

7. The device of any of the claims 4 to 6, wherein said processor (23) is adapted to provide the plurality of stimuli in accordance with a Posner cueing task and/or another attentional processing task, and to determine the at least one activity measure indicative of the brain activity in and/or between the at least one predetermined region of interest comprising one or more regions of the midcingulo-insular salience network so as to detect and/or quantify facilitated hypersensitivity by dysfunctional suppression of non-attended events and/or other dysfunctional attentional processing in the selection of, mental shifting between and/or continued focus maintenance on attended versus unattended events provided in said task.

8. The device of any of the claims 4 to 7, wherein said processor (23) is adapted to provide the plurality of stimuli in accordance with a cognitive processing task, and to determine the at least one activity measure indicative of the brain activity in and/or between the at least one predetermined region of interest comprising one or more regions of the frontoparietal and/or lateral fronto-parietal central executive network.

9. The device of any of the claims 4 to 8, wherein said processor (23) is adapted to provide the plurality of stimuli, comprising visual stimuli, and to determine the at least one activity measure indicative of the brain activity in and/or between the at least one predetermined region of interest comprising one or more regions of the visual cortex, the thalamus and/or other brain areas involved in the processing of visual information and/or vision, so as to detect abnormal activity in, and/or interaction within and/or between said one or more regions.

10. The device of any of the claims 4 to 9, wherein said processor (23) is adapted to provide different sets of sensory stimuli at different times and/or in different combinations during said acquisition, wherein said different sets correspond to different sensory stimulation conditions and/or stimulation characteristics, and wherein said processor is adapted to determine the at least one activity measure and/or the output value so as to score and/or rank the different types of sensory stimuli and/or thereto related stressors for their estimated potential to evoke, facilitate and/or trigger a migraine attack.

11. The device of any of the previous claims, wherein said processor (23) is adapted to receive, via said input (21), said functional brain imaging data comprising functional imaging data acquired in a resting state of the patient, without concomitant external stimulation, and to determine the at least one activity measure, or component thereof, by performing a resting-state functional connectivity imaging analysis so as to determine functional connectivity within and/or between said at least one predetermined region of interest, wherein the predetermined region of interest comprises one or more regions of the default mode network, the midcingulo-insular salience network and/or the fronto-parietal central executive network.

12. A magnetic resonance imaging system (100) comprising the device (122) of any of the previous claims configured so as to acquire and process functional magnetic resonance images of the patient's brain.

13. A method for analyzing neural activity in the brain of a patient by functional brain imaging to diagnose, assess, follow-up and/or classify a migraine condition of the patient, the method comprising:
acquiring (11) functional brain imaging data;
determining (14) at least one activity measure indicative of brain activity in and/or between at least one predetermined region of interest in the patient's brain based on said functional brain imaging data; and
determining and outputting (15) at least one value, based on said at least one activity measure, that is representative of a patient-specific migraine score indicating a probability and/or severity of the migraine, a migraine type classification, a prognostic value to predict frequency of migraine attacks and/or an estimated time until a next attack and/or a future evolution of the migraine condition, and/or an assessment of the influence of different types of migraine triggering events and/or of the predicted efficacy and/or suitability of different treatments.

14. The method of claim 13, wherein said acquiring (11) of the functional brain imaging data is repeated at different times, separated by at least a day or at least a month, so as to determine an evolution over time by the correspondingly determined activity measures and to determine at least one output value representative of changes in the migraine condition over time and/or of a future prediction of changes in the migraine condition.

15. A computer-program product adapted to perform the method of any of the claims 13 to 14 when executed by a computer.
